# EUROPEAN PATENT SPECIFICATION

(11) **EP 1 504 120 B1**
(45) Date of publication and mention of the grant of the patent: **19.08.2009**
(21) Application number: 03722865.7
(22) Date of filing: 14.05.2003
(51) Int. Cl.: C12Q 1/68

(54) **Methods for evaluating a disease condition by detection of particle and non-particle bound nucleic acids in blood plasma and serum**
Verfahren zur Beurteilung eines Krankheitzustandes mittels Nachweis von partikel- und nicht partikelgebundenen Nukleinsäuren in Blutplasma und -serum
Méthodes d'évaluation d'un état pathologique par la détection en sérum ou plasma de la fraction d'acides nucléiques liée aux particules et la fraction qui n'est pas liée

(30) Priority: 14.05.2002 US 380708 P
(43) Date of publication of application: 09.02.2005
(73) Proprietor: THE CHINESE UNIVERSITY OF HONG KONG, Shatin, New Territories (HK)
(72) Inventor: LO, Yuk Ming Dennis, Kowloon, Hong Kong (CN); NG, Kai On, New Territories, Hong Kong (CN); TSUI, Bo Yin, Kowloon, Hong Kong (CN); CHIU, Wai Kwun Rossa, New Territories, Hong Kong (CN); CHAN, Yuen Shan Lisa, New Territories, Hong Kong (CN); RAINER, Timothy Hudson, New Territories, Hong Kong (CN); LAM, Yuk Lan, Tseng Kwan O, Hong Kong (CN)
(74) Representative: Krauss, Jan
(86) International application number: PCT/GB2003/002081
(87) International publication number: WO 2003/095674

(56) References cited:
- WO-A-02/33120
- WO-A-97/35589
- CHIU R ET AL: "Effects of blood-processing protocols on fetal and total DNA quantification in maternal plasma" CLINICAL CHEMISTRY, vol. 47, no. 9, September 2001 (2001-09), pages 1607-13, XP002251902
- LO K-W ET AL: "Analysis of cell-free Epstein-Barr virus-associated RNA in the plasma of patients with nasopharyngeal carcinoma" CLINICAL CHEMISTRY, vol. 45, no. 8, August 1999 (1999-08), pages 1292-1294, XP002251903
- LO D ET AL: "Plasma DNA as a prognostic marker in trauma patients" CLINICAL CHEMISTRY, vol. 46, no. 3, March 2000 (2000-03), pages 319-23, XP002251721
- KOPRESKI M ET AL: "Detection of tumor messanger RNA in the serum of patients with malignant melanoma" CLINICAL CANCER RESEARCH, vol. 5, August 1999 (1999-08), pages 1961-65, XP002251904
- NG E ET AL: "Presence of filterable and nonfilterable mRNA in the plasma of cancer patients and healthy individuals" CLINICAL CHEMISTRY, vol. 48, no. 8, August 2002 (2002-08), pages 1212-17, XP002251905
- JERONIMO C ET AL: "MITOCHONDRIAL MUTATIONS IN EARLY STAGE PROSTATE CANCER AND BODILY FLUIDS" ONCOGENE, BASINGSTOKE, HANTS, GB, vol. 20, 23 August 2001 (2001-08-23), pages 5195-5198, XP009012216 ISSN: 0950-9232
- YAMAGATA M ET AL: "GLYCERALDEHYDE-3-PHOSPHATE DEHYDROGENASE mRNA EXPRESSION IN HEPATOCELLULAR CARCINOMA" INTERNATIONAL JOURNAL OF ONCOLOGY, EDITORIAL ACADEMY OF THE INTERNATIONAL JOURNAL OF ONCOLOGY,, GR, vol. 12, no. 5, 1998, pages 677-683, XP008020055 ISSN: 1019-6439

## Description

### FIELD OF THE INVENTION

This invention relates to the discovery that both non-particle and particle associated nucleic acids are present in blood plasma and serum and can be used to evaluate disease conditions.

### BACKGROUND OF THE INVENTION

New methods of simply and accurately evaluating disease conditions in patients are needed in order to aid in the detection, prognosis, diagnosis, monitoring and treatment of disease in patients worldwide.

It has recently been discovered that circulating nucleic acids in the plasma or serum of patients are associated with certain disease conditions (See, Lo YMD et al., N Eng J Med 1998;339:1734-8; Lo YMD, et al., Lancet 1997;350:485-7; Lo YMD, et al., Am J Hum Genet 1998;62:768-75; Chen XQ, et al., Nat Med 1996;2:1033-5, Nawroz H et al., Nat Med 1996;2:1035-7; Lo YMD et al., Lancet 1998;351:1329-30; Lo YMD, et al., Clin Chem 2000;46:319-23). Currently, little is known about the characteristics and biological origin of circulating nucleic acids. However, it is likely that cell death, including apoptosis, is one major factor (Fournie e al., Gerontology 1993;39:215-21; Fournie et al., Cancer Lett 1995;91:221-7.) Without being bound by theory, as cells undergoing apoptosis dispose nucleic acids into apoptotic bodies, it is possible that at least part of the circulating nucleic acids in the plasma or serum of human subjects is particle associated. In this application, it is demonstrated for the first time that circulating nucleic acids exist in both particle and non-particle associated form in the plasma and serum of human subjects. It is also demonstrated that by separating the circulating nucleic acids present in the plasma or serum of subjects into their particle associated and non-particle associated forms, disease conditions can be simply and accurately evaluated.

### BRIEF SUMMARY OF THE INVENTION

The present invention provides methods of evaluating a disease condition in a patient suspected of suffering or known to suffer from the disease condition as defined in claim 1. In one embodiment of the present invention, the invention includes obtaining a blood sample from the patient suspected of suffering or known to suffer from a disease condition, isolating plasma or serum from the blood sample, separating the plasma or serum into two or more fractions containing different relative concentrations of particle-associated and non-particle associated nucleic acid, and evaluating the disease condition by determining the amount or concentration or characteristic of nucleic acid in one or more fractions and comparing the amount or concentration or characteristic of nucleic acid in one or more fractions to a control.

In one aspect of the present invention, the nucleic acid is mRNA. In one embodiment the mRNA is *glyceraldehyde-3-phosphate dehydrogenase (GAPDH)* mRNA. In a second embodiment, the mRNA is *platelet basic protein (PBP)* mRNA. The mRNA may also be *human placental lactogen (hPL)* mRNA or the *beta-subunit of human chorionic gonadotropin (*β*hCG)* mRNA.

In a second aspect of the present invention, the nucleic acid is DNA. In one embodiment, the DNA is mitochondrial DNA.

In a third aspect of the present invention, a method of evaluating a disease condition in a patient suspected of suffering or known to suffer from the disease condition further comprises the step of amplifying the nucleic acid. In one embodiment, the nucleic acid is amplified by PCR. In a second embodiment, the nucleic acid is amplified by reverse-transcriptase PCR. In a third embodiment, the nucleic acid is amplified by real-time PCR. In a fourth embodiment, the nucleic acid is amplified by real-time reverse transcriptase PCR.

In a fourth aspect of the present invention, plasma or serum is separated into two or more fractions by filtration. In one embodiment, the filter size is about 5 µm or smaller. In a second embodiment, the filter size is about 0.22 µm. In a third embodiment, the filter size is about 0.45 µm.

In a fifth aspect of the present invention, plasma or serum is separated into two or more fractions by centrifugation. In one embodiment, the centrifugation is ultracentrifugation.

In a sixth aspect of the present invention, the disease condition to be evaluated is selected from the group consisting of cancer, stroke, and trauma. In one embodiment, the cancer is hepatocellular carcinoma or nasopharyngeal carcinoma.

In a seventh aspect of the present invention, the patient is pregnant and the method of evaluating a disease or physiologic condition in the patient or her fetus aids in the detection, monitoring, prognosis or treatment of the patient or her fetus.

The present invention also provides a method of evaluating the disease condition of a patient suspected of having or known to have or at risk of having hepatocellular carcinoma or a nasopharyngeal carcinoma. The method includes obtaining a sample of plasma or serum from the patient suspected of having or known to have hepatocellular carcinoma or a nasopharyngeal carcinoma, and detecting the quantity of glyceraldehyde-3-phosphate dehydrogenase (GAPDH) nucleic acid in the sample.

The present invention also provides a method of evaluating the disease condition of a patient suspected of having suffered from a trauma or known to have suffered from a trauma. The method includes obtaining a sample of plasma or serum from the patient suspected of having suffered from a trauma or known to have had suffered from a trauma, and detecting the quantity or concentration of mitochondrial nucleic acid in the sample.

As will be appreciated, the methods of the invention are be carried out, without an active step of obtaining a sample from the patient, on a sample previously obtained from the patient (e.g. a blood sample), or a sample isolated therefrom (e.g. a plasma or serum sample). Thus, the invention excludes any step performed directly on the patient.

As will also be appreciated, the various methods, aspects and embodiments of the invention may be combined, except where the context clearly requires otherwise.

### Definitions

The phrase "evaluating a disease condition" refers to assessing the disease condition of a patient. For example, evaluating the condition of a patient can include detecting the presence or absence of the disease in the patient. Once the presence of disease in the patient is detected, evaluating the disease condition of the patient may include determining the severity of disease in the patient. It may further include using that determination to make a disease prognosis, e.g. a life-span prediction or treatment plan. Evaluating the condition of a patient may also include detecting that a patient no longer has a disease condition but has suffered from the disease condition in the past. Evaluating the disease condition in that instant might also include determining the probability of reoccurrence of the disease condition or monitoring the reoccurrence in a patient. For example, detecting two acute ischemic events occurring within minutes, hours, or days of each other. Evaluating the disease condition might also include monitoring a patient for signs of disease. Evaluating a disease condition therefore includes detecting, diagnosing, or monitoring a disease condition in a patient as well as determining a patient prognosis or treatment plan. The method of evaluating a disease condition aids in risk stratification.

Blood plasma refers to the fraction of whole blood resulting from centrifugation of blood treated with anticoagulants. Blood serum refers to the watery portion of fluid remaining after a blood sample has coagulated.

As used in the present invention, the phrase "comparing the amount or concentration or characteristic of nucleic acid in one or more fractions to a control" is equivalent to comparing the amount or concentration or characteristic of nucleic acid in one or more fractions to a standard. Circulating nucleic acids exist in the blood plasma or serum of both healthy patients and disease patients in both particle-associated and non-particle associated forms. By comparing the relative concentration of particle-associated and non-particle associated nucleic acid in a patient suspected of having a disease, known to have a disease or at risk of having a disease to the relative concentration of particle-associated and non-particle associated nucleic acid in a healthy subject, in a sample taken from the patient at an earlier time, or in a sample taken from another diseased individual, it is possible to evaluate disease conditions. By making inter-fraction comparisons, it is also possible to evaluate disease conditions. In some embodiments of the invention, the control might not be a second sample but instead an average of data from a variety of persons who are classified as healthy or as suffering from various disease conditions, e.g., trauma and cancer. The data collected may correspond various levels or concentrations of non-particle associated nucleic acid in the blood to severity, prognosis or diagnosis of disease. A skilled practitioner can compare the amount or concentration of non-particle associated nucleic acid in fraction A of an individual to a control and determine the presence or absence of disease in the individual from whom which the sample was obtained. The skilled practitioner can also use the comparison to determine disease type or stage. The skilled practitioner might also use the comparison to determine disease severity or predict patient life-span. The skilled practitioner can use the comparison to aid in risk stratification, monitoring or treatment of the disease condition in a patient.

Circulating nucleic acid present in the blood plasma or serum may exist in two forms, "particle-associated" and "non-particle associated". Particle-associated nucleic acids are defined as nucleic acids that are contained inside or adhered to or linked to or on the surface of particles. The term "particle" is defined as any material that can be demonstrated by physical means to have a finite size from 0.1 µm to 5 µm in diameter or one that is pelletable by ultracentrifugation following prior filtration using a 5 µm filter. Sizes larger than 5 µm in diameter will include intact cells and so are excluded from our invention. Physical means used to determine the size of particle-associated nucleic acids include, but are not limited to, centrifugation, ultracentrifugation, sedimentation, filtration, optical microscopy or electron microscopy. Accordingly, the term "particle-associated nucleic acids" includes both extracellular and intracellular nucleic acids present in the blood plasma or serum providing that the nucleic acids are bound to a particle from 0.1 µm to 5 µm in size or one which is pelletable by ultracentrifugation. For example, extracellular nucleic acids may be complexed to ribosomes, lipids or proteolipids. They may be protein-bound or within apoptotic bodies. The term "particle associated nucleic acids" may also include those extracellular nucleic acids complexed to ribosomes, lipids or proteolipids. For the purposes of the present invention, nucleic acids within apopotic bodies are included within the term "particle-associated nucleic acid" as long as the bound nucleic acid complex is from 0.1 µm to 5 µm in size. Accordingly, for the purposes of the present invention, particle-associated nucleic acids are those nucleic acids circulating in the blood stream that are within cell remnants (including but not limited to platelets which are liberated by megakaryocytes), complexed with cellular remnants, or associated with organelles such as but not limited to mitochondria, or associated with apoptotic bodies, or bound to other particles from 0.1 µm to 5 µm in size. Non-particle associated nucleic acids are circulating nucleic acids that are not complexed to cellular remnants or apoptotic bodies but may be complexed to particles smaller than about 0.1 µm in size. For the purpose of this invention, non-particle associated nucleic acids also include those circulating nucleic acids that are not pelletable by ultracentrifugation following filtration through a 5 µm filter.

"Nucleic acid" refers to a deoxyribonucleotide or ribonucleotide polymer in either single- or double-stranded form, and unless otherwise limited, would encompass known analogs of natural nucleotides that can function in a similar manner as naturally occurring nucleotides.

The phrase "a sample of blood plasma or serum", as used herein, refers to a sample of blood plasma or serum obtained from a subject. Frequently the sample will be a "clinical sample" which is a sample derived from a patient with a disease or suspected of having a disease or a physiological condition needing medical attention such as pregnancy (a "patient"). The sample as initially obtained from the patient may contain additional components other than blood plasma or serum. For example, the sample may initially be a sample of whole blood purified to its plasma or serum components. Either "fresh" blood plasma or serum, or frozen (stored) and subsequently thawed plasma or serum may be used for the methods of this invention. Frozen (stored) plasma or serum should optimally be maintained at storage conditions of -20 to -70 degrees centigrade until thawed and used.

The terms "hybridize(s) specifically" or "specifically hybridize(s)" refer to complementary hybridization between an oligonucleotide (*e*.*g*., a primer or labeled probe) and a target sequence. The term specifically embraces minor mismatches that can be accommodated by reducing the stringency of the hybridization conditions to achieve the desired priming for the PCR polymerases or detection of hybridization signal.

The term "oligonucleotide" refers to a molecule comprised of two or more deoxyribonucleotides or ribonucleotides, such as primers, probes, and other nucleic acid fragments. The exact size of an oligonucleotide depends on many factors and the ultimate function or use of the oligonucleotide. "Adding" an oligonucleotide refers to joining an oligonucleotide to another nucleic acid molecule. Typically, adding the oligonucleotide is performed by ligating the oligonucleotide using a DNA ligase.

The term "primer" refers to an oligonucleotide, whether natural or synthetic, capable of acting as a point of initiation of DNA synthesis under conditions in which synthesis of a primer extension product complementary to a nucleic acid strand is induced, *i*.*e*., in the presence of four different nucleoside triphosphates and an agent for polymerization (such as DNA polymerase or reverse transcriptase) in an appropriate buffer and at a suitable temperature. A primer is preferably a single-stranded oligodeoxyribonucleotide sequence. The appropriate length of a primer depends on the intended use of the primer but typically ranges from about 15 to about 30 nucleotides. Short primer molecules generally require cooler temperatures to form sufficiently stable hybrid complexes with the template. A primer need not reflect the exact sequence of the template but must be sufficiently complementary to specifically hybridize with a template.

"Probe" refers to an oligonucleotide which binds through complementary base pairing to a subsequence of a target nucleic acid. It will be understood by those skilled in the art that probes will typically substantially bind target sequences lacking complete complementarity with the probe sequence depending upon the stringency of the hybridization conditions. The probes are typically directly labeled (*e*.*g*., with isotopes or fluorescent moieties) or indirectly labeled such as with digoxigenin or biotin. By assaying for the presence or absence of the probe, one can detect the presence or absence of the target.

The nucleic acids may be single stranded or double stranded, as specified, or contain portions of both double stranded or single stranded sequence. The depiction of a single strand also defines the sequence of the complementary strand; thus the sequences described herein also provide the complement of the sequence. The nucleic acid may be DNA, both genomic and cDNA, RNA or a hybrid, where the nucleic acid may contain combinations of deoxyribo- and ribo-nucleotides, and combinations of bases, including uracil, adenine, thymine, cytosine, guanine, inosine, xanthine, hypoxanthine, isocytosine, isoguanine, *etc.* "Transcript" typically refers to a naturally occurring RNA, e.g., a pre-mRNA, hnRNA, or mRNA. As used herein, the term "nucleoside" includes nucleotides and nucleoside and nucleotide analogs, and modified nucleosides such as amino modified nucleosides. In addition, "nucleoside" includes non-naturally occurring analog structures. Thus, *e*.*g*. the individual units of a peptide nucleic acid, each containing a base, are referred to herein as a nucleoside.

The phrase "selectively (or specifically) hybridizes to" refers to the binding, duplexing, or hybridizing of a molecule only to a particular nucleotide sequence under stringent hybridization conditions when that sequence is present in a complex mixture (*e*.*g*., total cellular or library DNA or RNA).

The term "substantially identical" indicates that two or more nucleotide sequences share a majority of their sequences. Generally, this will be at least about 90% of their sequences and preferably about 95% of their sequences. Another indication that the sequences are substantially identical is if they hybridize to the same nucleotide sequence under stringent conditions (*see, e*.*g*., Sambrook and Russell, eds, Molecular Cloning: A Laboratory Manual, 3rd Ed, vols. 1-3, Cold Spring Harbor Laboratory Press, 2001; and Current Protocols in Molecular Biology, Ausubel, ed. John Wiley & Sons, Inc. New York, 1997). Stringent conditions are sequence-dependent and will be different in different circumstances. Generally, stringent conditions are selected to be about 5°C (or less) lower than the thermal melting point (Tₘ) for the specific sequence at a defined ionic strength and pH. The Tₘ of a DNA duplex is defined as the temperature at which 50% of the nucleotides are paired and corresponds to the midpoint of the spectroscopic hyperchromic absorbance shift during DNA melting. The Tₘ indicates the transition from double helical to random coil

Typically, 'stringent conditions' will be those in which the salt concentration is about 0.2xSSC at pH 7 and the temperature is at least about 60°C. For example, a nucleic acid can be identified in standard filter hybridizations using the nucleic acids disclosed here under stringent conditions, which for purposes of this disclosure, include at least one wash (usually two) in 0.2x SSC at a temperature of at least about 60°C, usually about 65°C, sometimes 70°C for 20 minutes, or equivalent conditions. For polymerase chain reaction (PCR), an annealing temperature of about 5°C below Tₘ, is typical for low stringency amplification, although annealing temperatures may vary between about 32°C and 72°C, *e*.*g*., 40°C, 42°C, 45°C, 52°C, 55°C, 57°C, or 62°C, depending on primer length and nucleotide composition. or high stringency PCR amplification, a temperature at, or slightly (up to 5°C) above, primer Tₘ is typical, although high stringency annealing temperatures can range from about 50°C to about 72°C, and are often 72°C, depending on the primer and buffer conditions (Ahsen et al., Clin. Chem. 47:1956-61, 2001). Typical cycle conditions for both high and low stringency amplifications include a denaturation phase of 90°C-95°C for 30 sec.-2 min., an annealing phase lasting 30 sec.-2 min., and an extension phase of about 72°C for 1-6 min.

The terms "identical" or "percent identity", in the context of two or more nucleic acids, refer to two or more sequences or subsequences that are the same or have a specified percentage of nucleotides that are the same (i.e., about 70% identity, preferably 75%, 80%, 85%, 90%, or 95% identity over a specified region, when compared and aligned for maximum correspondence over a comparison window, or designated region as measured using BLAST or BLAST 2.0 sequence comparison algorithms with default parameters described below, or by manual alignment and visual inspection. Such sequences are then said to be "substantially identical." This definition also refers to the complement of a test sequence. Preferably, the identity exists over a region that is at least about 15, 20 or 25 nucleotides in length, or more preferably over a region that is 50-100 nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 15 to 600, (usually about 20 to about 200, or more usually about 50 to about 150) in which a sequence may be compared to a reference sequence of the same number of contiguous positions after the two sequences are optimally aligned. Methods of alignment of sequences for comparison are well-known in the art. Optimal alignment of sequences for comparison can be conducted, *e*.*g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the default parameters described herein, to determine percent sequence identity for the nucleic acids described herein. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=-4 and a comparison of both strands.

The BLAST algorithm also performs a statistical analysis of the similarity between two sequences (*see*, *e*.*g*., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

### BRIEF DESCRIPTION OF THE DRAWINGS

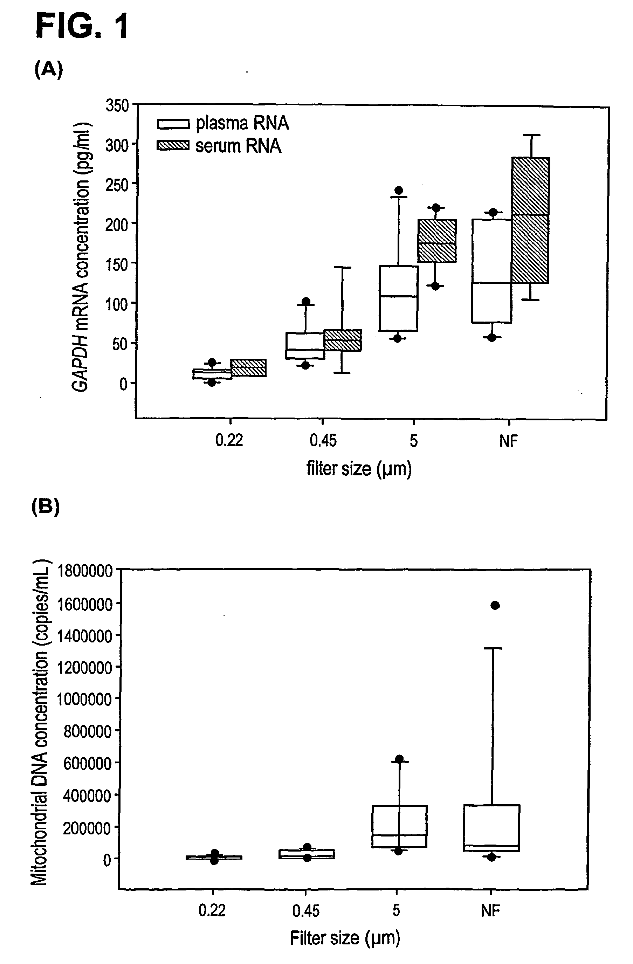

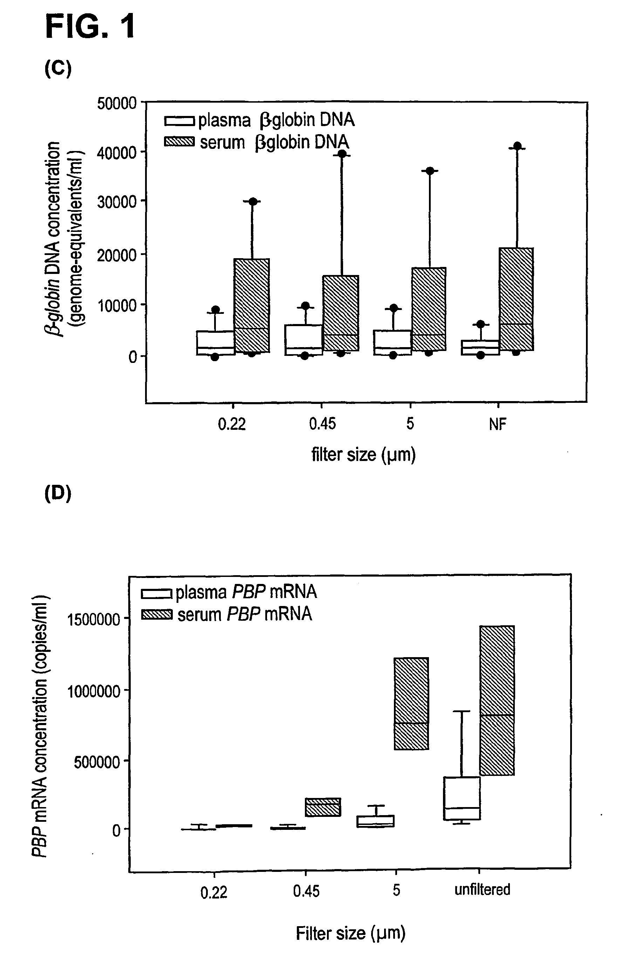

**Fig. 1****.** Plasma/serum mRNA and DNA concentrations of healthy subjects following different pore-sized filtration treatments: ***A***, plasma/serum *GAPDH* mRNA concentrations (pg/ml) as determined by real-time quantitative RT-PCR (*Y axis*) are plotted against the processing categories (*X axis*); ***B***, plasma mitochondrial DNA concentrations (copies/ml) as determined by real-time quantitative PCR (*Y axis*) are plotted against the processing categories (*X axis*); ***C***, plasma/serum β*-globin* DNA concentrations (genome-equivalents/ml) as determined by real-time quantitative PCR (*Y axis*) are plotted against the processing categories (*X axis*); ***D**,* plasma/serum *PBP* mRNA concentrations (copies/ml) as determined by real-time quantitative RT-PCR (*Y axis*) are plotted against the processing categories (*X axis*). The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

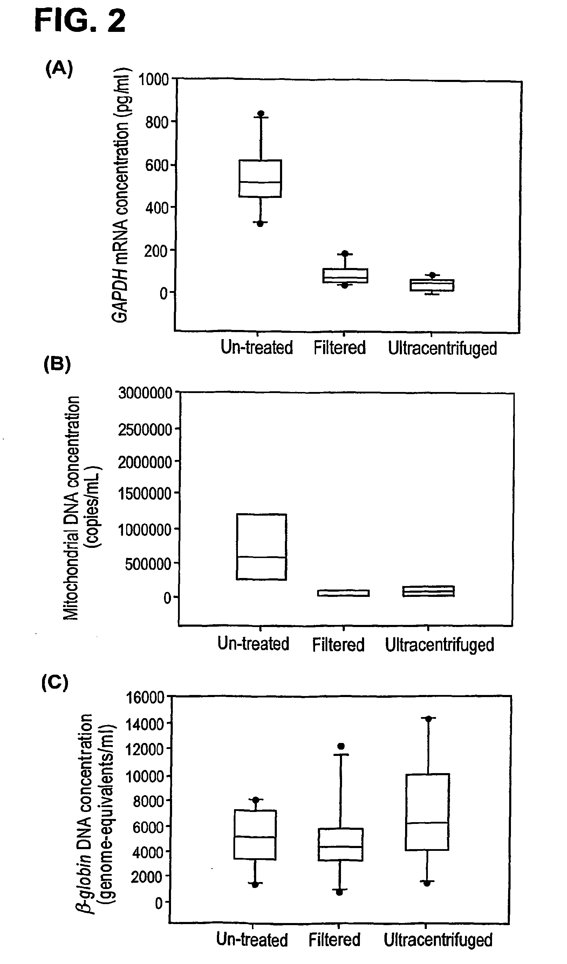

**Fig. 2****.** Plasma mRNA and DNA concentrations of healthy subjects following filtration and ultracentrifugation treatments: ***A***, plasma *GAPDH* mRNA concentrations (pg/ml) as determined by real-time quantitative RT-PCR (*Y axis*) are plotted against the processing categories (*X axis*); ***B**,* plasma mitochondrial DNA concentrations (copies/ml) as determined by real-time quantitative PCR (*Y axis*) are plotted against the processing categories (*X axis*); ***C***, plasma β*-globin* DNA concentrations (genome-equivalents/ml) as determined by real-time quantitative PCR *(Y axis*) are plotted against the processing categories (*X axis*). *Um-treated*, no treatment; *Filtered*, 0.22-µm filtration; *Ultracentrifuged,* ultracentrifugation at 99,960 X g. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

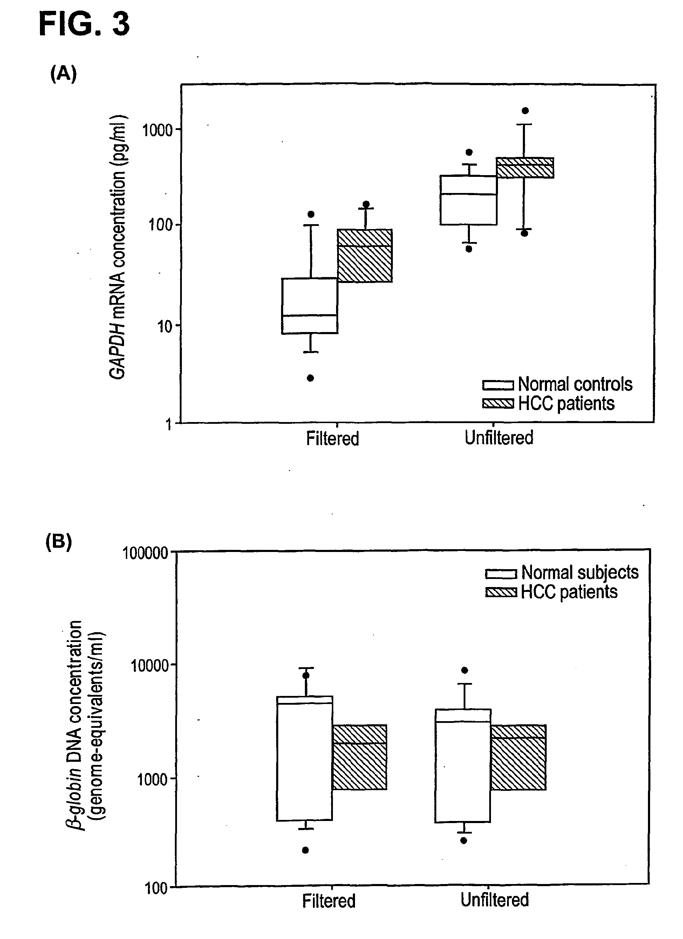

**Fig. 3****.** Comparisons of plasma mRNA and DNA concentrations in healthy subjects and hepatocellular carcinoma (HCC) patients with or without 0.22-µm filtration treatments. The processing categories are shown on the *X axis. **A***, Plasma *GAPDH* mRNA concentrations (pg/ml) as determined by real-time quantitative RT-PCR are plotted on the *Y axis* (common logarithmic scale). ***B***, Plasma β*-globin* DNA concentrations (genome-equivalents/ml) as determined by real-time quantitative PCR are plotted on the *Y axis* (common logarithmic scale). ▭ denotes healthy subjects while 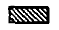 denotes HCC patients. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

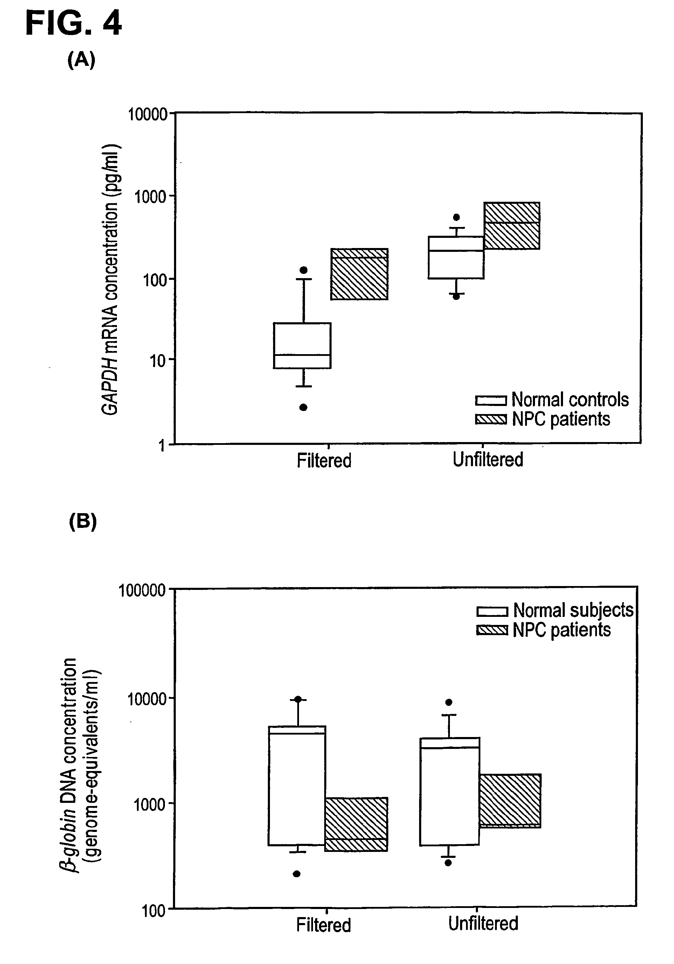

**Fig. 4****.** Comparisons of plasma mRNA and DNA concentrations in healthy subjects and nasopharyngeal carcinoma (NPC) patients with or without 0.22-µm filtration treatments. The processing categories are shown on the *X axis. **A**,* Plasma *GAPDH* mRNA concentrations (pg/ml) as determined by real-time quantitative RT-PCR are plotted on the *Y axis* (common logarithmic scale). ***B***, Plasma β*-globin* DNA concentrations (genome-equivalents/ml) as determined by real-time quantitative PCR are plotted on the *Y axis* (common logarithmic scale). ▭ denotes healthy subjects while 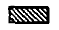 denotes NPC patients. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

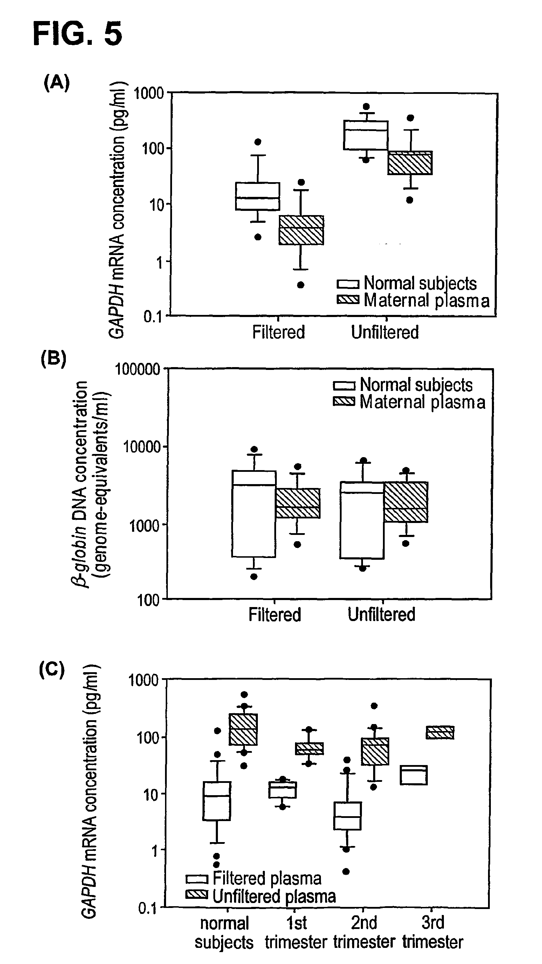

**Fig. 5****.** Comparisons of plasma mRNA and DNA concentrations in healthy subjects and pregnant women with or without 0.22-µm filtration treatments. The processing categories are shown on the *X axis. **A**,* Plasma *GAPDH* mRNA concentrations (pg/ml) as determined by real-time quantitative RT-PCR are plotted on the *Y axis* (common logarithmic scale). ***B**,* Plasma β*-globin* DNA concentrations (genome-equivalents/ml) as determined by real-time quantitative PCR are plotted on the *Y axis* (common logarithmic scale). ***C***, Plasma mRNA concentrations of healthy subjects and pregnant women of various stages of gestation. Plasma *GAPDH* mRNA concentrations (pg/ml) as determined by real-time quantitative RT-PCR (*Y* axis, common logarithmic scale) are plotted against the categories (normal subjects and pregnant women from first, second and third trimesters) (*X axis*). ▭ denotes filtered plasma while 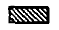 denotes unfiltered plasma. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

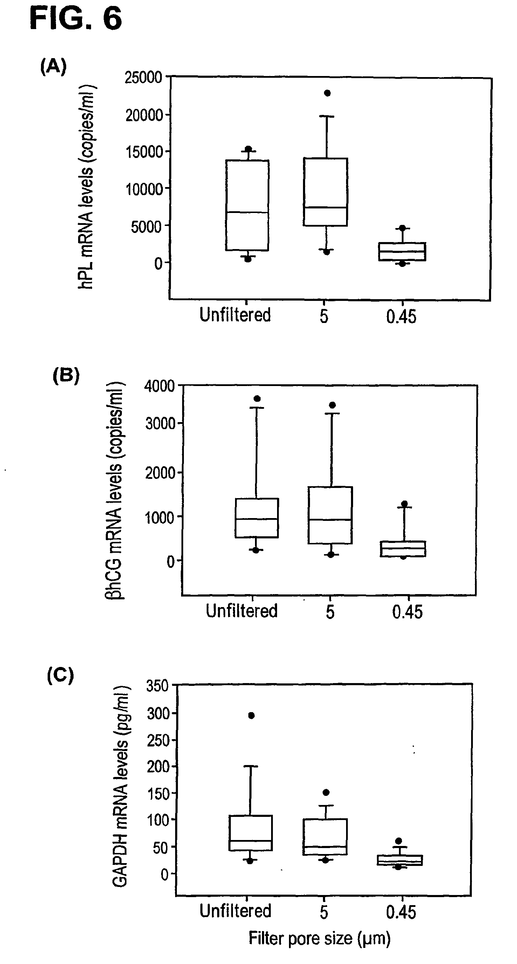

**Fig. 6****.** Concentrations of placental-derived mRNA in plasma of pregnant women following different pore-sized filtration treatments. The processing categories are shown on the *X axis. **A***, plasma *hPL* mRNA concentrations (copies/ml) as determined by real-time quantitative RT-PCR are plotted on the *Y axis. **B**,* plasma β*hCG* mRNA concentrations as determined by real-time quantitative RT-PCR (copies/ml) are plotted on the *Y axis. **C***, plasma *GAPDH* mRNA concentrations as determined by real-time quantitative RT-PCR (pg/ml) are plotted on the *Y axis*. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

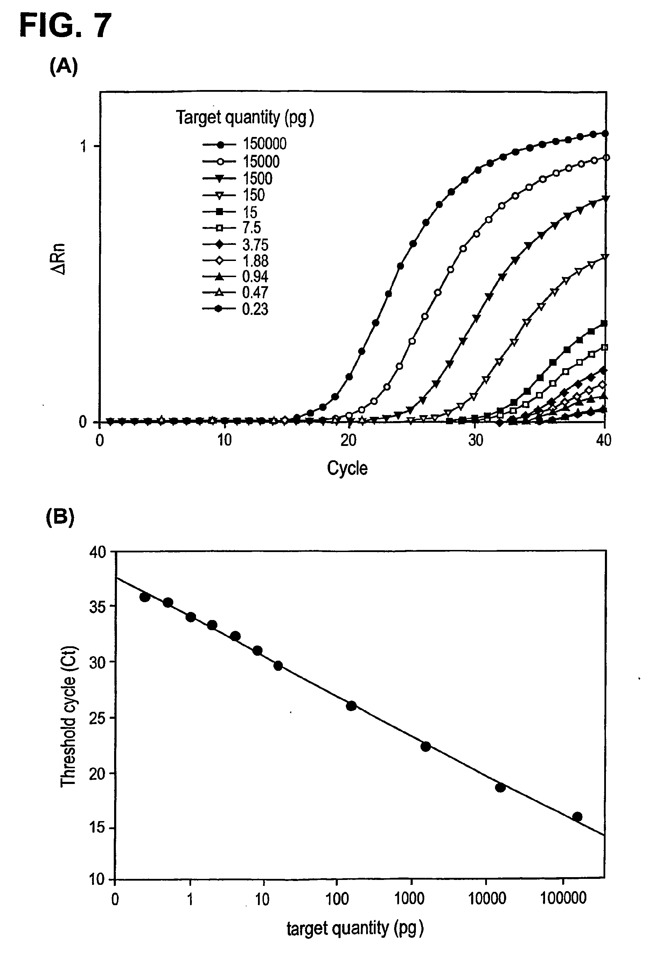

**Fig. 7****.** Detection of *GAPDH* mRNA by real-time quantitative RT-PCR. ***A***, amplification plot of fluorescence intensity over the background (*Y axis*) against the RT-PCR cycle (*X axis*). Each plot corresponds to a particular input target quantity marked by a corresponding symbol. ***B***, plot of the threshold cycle (C_{T}) (*Y axis*) against the input target quantity (*X axis,* common logarithmic scale). The input target quantity was expressed as pg of human control RNA.

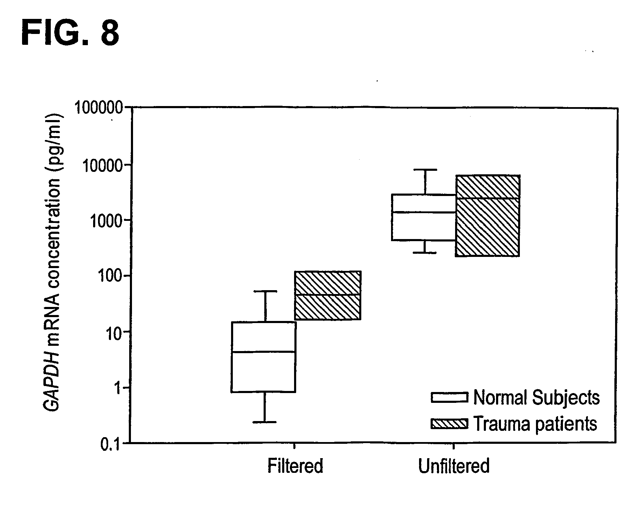

**Fig. 8****.** Comparisons of plasma mRNA concentrations in healthy subjects and trauma patients with or without 0.22-µm filtration treatments. Plasma *GAPDH* mRNA concentrations (pg/ml) as determined by real-time quantitative RT-PCR (*Y axis*, common logarithmic scale) are plotted against the processing categories *(X axis).* ▭ denotes the healthy subjects while 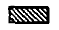 denotes the trauma patients. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

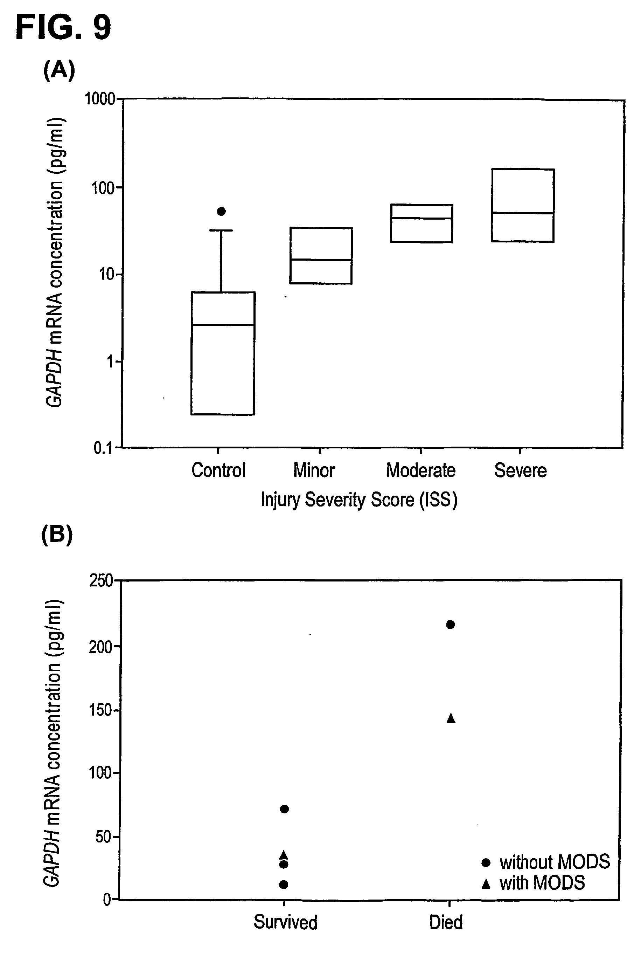

**Fig. 9****.** Comparisons of plasma mRNA concentrations in control subjects and trauma patients of different levels of severity with 0.22-µm filtration treatment. ***A*,** plasma *GAPDH* mRNA concentrations (pg/ml) in the filtered plasma samples as determined by real-time quantitative RT-PCR (*Y axis*, common logarithmic scale) are plotted against the categories (healthy control subjects and trauma patients with different severity levels) (*X axis*). The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles. ***B***, 0.22-µm filtered plasma *GAPDH* mRNA concentrations of trauma patients with major injury. Plasma *GAPDH* mRNA concentration (pg/ml) as determined by real-time quantitative RT-PCR *(Y axis)* are plotted against the categories (survived and died) *(X axis)*. • denotes patients without developed MODS while ▲ denotes patients with developed MODS.

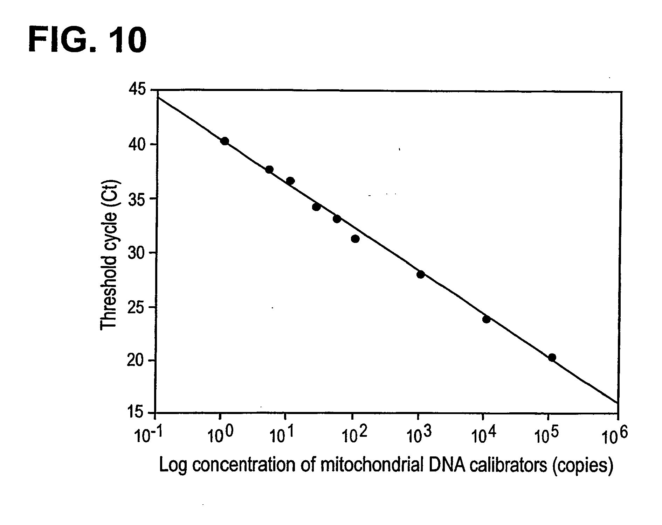

**Fig. 10****.** Calibration curve for quantitative analysis of mitochondrial DNA by real-time quantitative PCR. The threshold cycle (C_{T}) (*Y axis*) are plotted against the input target quantity (*X axis*, common logarithmic scale). The input target quantity was express as copies of mitochondrial DNA amplicon.

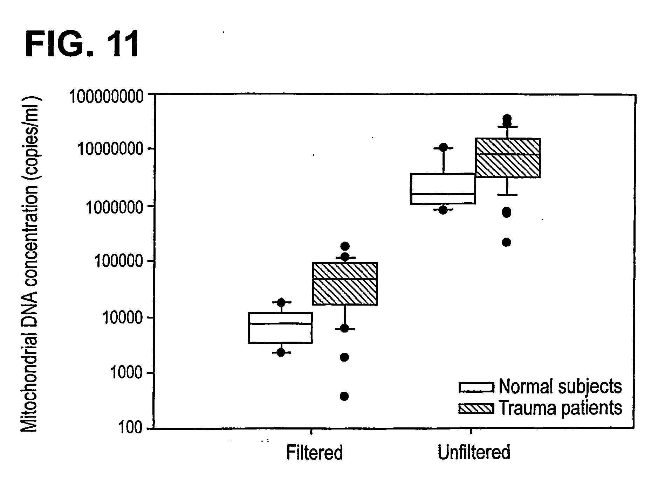

**Fig. 11****.** Comparisons of plasma mitochondrial DNA concentrations in healthy subjects and trauma patients with or without 0.22-µm filtration treatments. The processing categories are shown on the *X axis*. Plasma mitochondrial DNA concentrations (copies/ml) as determined by real-time quantitative PCR are plotted on the *Y axis* (common logarithmic scale). ▭ denotes the healthy subjects while 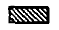 denotes the trauma patients. The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

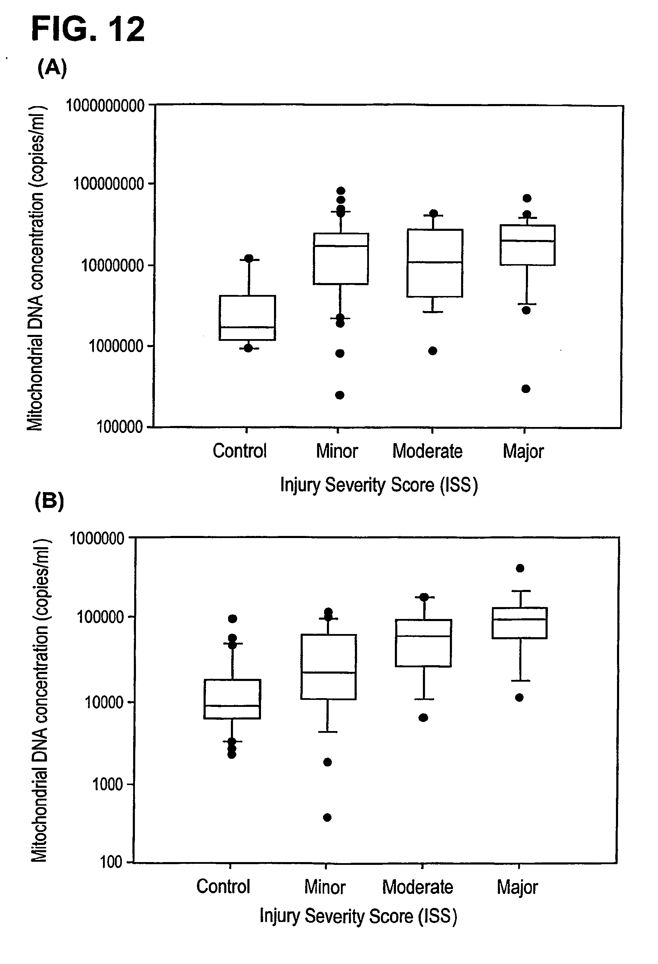

**Fig. 12****.** Comparisons of plasma mitochondrial DNA concentrations in healthy subjects and trauma patients of different levels of severity with or without 0.22-µm filtration treatments. The categories (healthy control subjects and trauma patients of different levels of severity) are shown on the *X axis. **A,*** plasma mitochondrial DNA concentrations (copies/ml) of the unfiltered plasma samples as determined by real-time quantitative PCR are plotted on the *Y axis* (common logarithmic scale). ***B*,** plasma mitochondrial DNA concentrations (copies/ml) of the filtered plasma samples as determined by real-time quantitative PCR are plotted on the *Y axis* (common logarithmic scale). The *lines inside the boxes* denote the medians. The *boxes* mark the interval between the 25^{th} and 75^{th} percentiles. The *whiskers* denote the interval between the 10^{th} and 90^{th} percentiles. The *filled circles* mark the data points outside the 10^{th} and 90^{th} percentiles.

### DETAILED DESCRIPTION OF THE INVENTION

This invention pertains to the surprising discovery that circulating nucleic acids exist in both particle associated and non-particle associated forms in the plasma or serum of healthy subjects and diseased patients and can be used to evaluate disease conditions. In one aspect of the invention, after separating blood plasma or serum into two or more fractions containing different relative concentrations of particle-associated and non-particle associated nucleic acids, followed by extraction of nucleic acids from these fractions, comparisons can be made between the relative concentrations of particle associated and non-particle associated nucleic acids present in a blood plasma or serum sample from a patient and from a control. The difference in concentration between the two samples can be used to further evaluate the disease condition. The methods of the present invention also provide for the detection and quantification of nucleic acids in the plasma or serum of a pregnant woman for the evaluation of a disease or condition in either her or her fetus.

It is particularly surprising that the separation of nucleic acids circulating in the blood plasma or serum of a patient into fractions can be used to aid in the diagnosis, detection, treatment, prognosis and monitoring of disease conditions. Although it has been found that increased levels of total nucleic acids may be indicative of certain diseases, it has never been demonstrated that by separating circulating nucleic acids into fractions with differing concentrations of particle-associated and non-particle associated nucleic acids, a disease condition can be further evaluated. In particular, it has never been shown that for those disease conditions where the total level of circulating nucleic acids is not indicative of disease, the relative concentration of nucleic acid in a non-particle associated fraction can be indicative of disease and used to further evaluate disease.

### Selecting a patient population

The present invention provides methods for evaluating a disease condition in a patient suspected of suffering, known to suffer or at risk of suffering from the disease condition. A disease condition includes any disease condition marked by an increase in non-particle associated and particle-associated nucleic acids circulating in the blood plasma or serum of the individual. Diseases marked by an increased concentration of non-particle associated nucleic acids or particle-associated nucleic acids circulating in the blood plasma or serum of the individual include but are not limited to diseases characterized by abnormal levels of cell death, for example, diseases associated with the inappropriate activation of apoptosis. The inappropriate activation of apoptosis can contribute to a variety of pathological diseases states including, but not limited to, for example, the acquired immunodeficiency syndrome (AIDS), neurodegenerative diseases, preeclampsia, Alzheimer's disease and ischemic injuries. Conditions such as cancer, preeclampsia, trauma, myocardical infarction, stroke and ageing can be characterized by abnormal levels of cell death. Standard methods, e.g., clinical examination, laboratory workups, are used to determine if a patient is suffering from or at risk of suffering from a disease condition associated with abnormal levels of cell death, e.g., AIDS, neurodegeneative disorders, ischemic injuries, trauma, cancer and the like.

The present invention also provides methods for evaluating a disease condition in a pregnant woman or in her fetus. The determination that a woman is pregnant can be done by standard techniques known in the art.

The present invention provides methods for evaluating a disease condition in a patient suspected of having, known to have or at risk of having hepatocellular carcinoma or nasopharyngeal carcinoma. Patients with hepatocellular carcinoma or nasopharyngeal carcinoma may have a spectrum of pathology identifiable by a skilled practitioner. The diagnosis of hepatocellular carcinoma or nasopharyngeal carcinoma may be made based on a clinical, radiological, endoscopic or laboratory workup.

The present invention provides methods for evaluating trauma in a patient. Trauma is a world-wide problem that claims millions of lives yearly. Late deaths corresponding to those who die days or weeks after traumatic injury commonly occur as a result of sepsis or multiple organ dysfunction syndrome (MODS). It is believed that an initial insult triggers systemic inflammatory response after severe trauma and subsequent insult, e.g., aggressive resuscitation, surgery, or sepsis, eventually leads to organ failure involving the lungs, liver, kidneys, brain, gastrointestinal and hematological systems. Anti-inflammatory interventions have frequently produced disappointing results in terms of survival rates of patients. One possible reason is the inability to accurately identify traumatic patients at high-risk at an early stage so that potentially effective interventions may be applied to prevent the later development of MODS. The present invention provides methods for evaluating a disease condition in a patient suspected of having, known to have or at risk of having trauma. Using standard clinical, radiological or laboratory workups known in the art, a practitioner will make an initial determination that a patient is suffering from or at risk of suffering from trauma and will use the methods of the present invention to further evaluate the traumatic condition.

### Obtaining blood samples

Blood can be drawn by standard methods into a collection tube, e.g., siliconized glass tube, either without anticoagulation for the preparation of serum, or with anticoagulants, e.g., EDTA, sodium citrate, or heparin. In a preferred embodiment, fractionation of plasma or serum from whole blood is performed prior to freezing. Fractionation of fresh plasma can be done by standard methods, for example, fresh plasma or serum may be fractionated from whole blood by centrifugation according to known methods. In a preferred embodiment, centrifugation is gentle so that it does not fraction out apoptotic bodies from the plasma or serum. For some embodiments of the present invention, e.g., amplification of RNA with RT-PCR, it is preferable to pretreat heparinized blood with heparinase.

### Nucleic acid extraction

Nucleic acid can be extracted from blood plasma or serum using standard extraction methods known in the art, e.g., gelatin extraction, silica, glass bead or diatom extraction, guanidine or guanidinium-based extraction, chemical extraction methods, or size exclusion or anion exchange chromatographic methods (See U.S. Patent 6,329,179 and International Publication Number WO 01/42504). Nucleic acid, e.g., DNA, can also be extracted using a QIAamp Blood Kit according to the "blood and body fluid protocol" as recommended by the manufacturer (Chen XQ, et al. Nat. Med. 1996;2:1033-5).

In one method, nucleic acid is precipitated from plasma or serum with gelatin by a method modified from that of Fournie et al. (1986 Anal. Biochem. 158 250-256). A gelatin solution is prepared by mixing gelatin with water, autoclaving the mixture and filtering it through a 0.2 micron filter. The resultant solution is sequentially frozen in a dry ice/ethanol bath and thawed at room temperature. A 0.3% gelatin solution is prepared using the resultant solution. Blood plasma or serum is mixed with EDTA, sterile water, and emulsified. After centrifugation, the aqueous layer is removed and transferred to a clean tube. DNA is precipitated by adding the 0.3% gelatin solution and ethanol, followed by incubation at -20°C.

In another method, nucleic acid is extracted in an enriching method, or extracted nucleic acid is further enriched, using probe specific hybridization wherein said hybridizing probes are immobilized to a substrate, e.g., nylon or magnetic beads, from which contaminating species, e.g., unwanted nucleic acid, can be removed using known methods, *e*.*g*., stringent washings. Other extraction methods include using a magnetic or electric field.

In yet another method, nucleic acid can be extracted from blood serum or plasma by heating the serum or plasma, at a temperature from about 90°C to about 100°C for up to about 20 minutes.

Circulating nucleic acids can be extracted form plasma or serum using glass beads, silica particles or diatom as in the methods or adapted methods of Boom *et al.* (Boom et al., 1991, J. Clin. Microbiol. 29:1804-1811; Boom et al. 1989, J. Clin. Microbiol. 28:495-503). Blood plasma or serum is mixed with a silica suspension made by known methods. The mixture is then centrifuged and the supernatant is aspirated and discarded. After washing the silica-DNA pellets with washing buffer, ethanol and acetone, it is dried and then the sample is eluted with a TE buffer with or without Proteinase K. Following elution, the sample is centrifuged and the DNA-containing supernatant recovered.

The skilled practitioner will know how to extract DNA or RNA from blood plasma or serum using other known methods. Any means of purifying DNA or RNA from blood plasma or serum can be used in the methods of the present invention.

### The separation of blood plasma or serum nucleic acids into two or more fractions

Nucleic acids are extracted from blood plasma or serum following the separation of a blood plasma or serum sample into two or more fractions containing different relative concentrations of particle-asssociated and non-particle associated nucleic acid. Before separation, the plasma or serum sample will contain both particle-associated and non-particle associated species. After separation, these fractions will contain different relative concentrations of particle-associated and non-particle-associated nucleic acids. Separation of the blood plasma or serum sample into these fractions can be by any separation technique, including those that can separate particles in terms of their sizes or to their behavior following ultracentrifugation.

In one method, the extracted nucleic acids are separated by filtration with different pore-sized filters, e.g., 5 µm, 0.45 µm or 0.22 µm filter size. Particle-associated nucleic acids, e.g., nucleic acids within apoptotic bodies of a certain size, will be unable to pass through a filter with a pore size below the certain size, whereas non-particle associated nucleic acids or particle-associated nucleic acids associated with particles below the certain size will pass through. For illustrative purposes, if a filter size of 0.22 µm is used on a blood plasma or serum sample containing nucleic acids associated with particles ranging from 0.1 µm to 5 µm (particle-associated nucleic acids), the fluid that passes through the filter will contain a lower concentration of particle-associated nucleic acids than a blood plasma or serum sample that has been passed through a 0.45 µm filter.

In another method, intensive centrifugation forces or ultracentrifugation is used to pellet the particle-associated nucleic acids from a blood plasma or serum sample. In the methods of the present invention, centrifugation forces capable of separating the circulating nucleic acids into a particle-associated and non-particle associated fraction are large enough to pellet virtually all particulate matter present in the sample. Centrifugal forces of 70,000g to 100,000g can be used to convert a plasma or serum sample initially containing both particle-associated and non-particle-associated nucleic acids, into one in which the fractional concentration of non-particle-associated nucleic acids is increased.

Any method of separating particle-associated nucleic acids into different size species can be used in the methods of the present invention. Other methods are well known in the art and include fluorescence activated cell sorting, magnetic activated cell sorting, or differential sedimentation.

### Nucleic acid detection methods

The nucleic acids detected in the methods of the invention are typically from about 40 nucleotides in length to several thousand nucleotides in length. Usually, the nucleic acids are from about 80 to about 200 nucleotides.

After nucleic acid, e.g., DNA or RNA, has been isolated from blood plasma or serum, the nucleic acids are detected using methods known in the art. The nucleic acids can be detected after separation into a non-particle associated and a particle-associated fraction. Any of the conventional DNA or RNA detection methods can be used for the detection and quantification, e.g., amount or concentration, of nucleic acid. In a preferred embodiment, any means for detecting low copy number nucleic acids are used to detect the nucleic acids of the present invention. Means for detecting and quantifying low copy number nucleic acids include analytic biochemical methods such as electrophoresis, capillary electrophoresis, high performance liquid chromatography (HPLC), thin layer chromatography (TLC), hyperdiffusion chromatography, mass spectroscopy and the like. These methods are well known in the art and are thus not described in detail (See for example, U.S. Patent Nos. 6,013,422, 6,261,781, 6,268,146, or 5,885,775).

The methods of the present invention typically but not always rely on amplification or signal amplification methods for the detection of the nucleic acids. One of skill will recognize that amplification of target sequences in a sample may be accomplished by any known method, such as ligase chain reaction (LCR), Qβ-replicase amplification, transcription amplification, and self-sustained sequence replication, each of which provides sufficient amplification.

The PCR process is well known in the art. For a review of PCR methods and protocols, *see*, *e.g.,* Innis, et al. eds. PCR Protocols. A Guide to Methods and Application (Academic Press, Inc., San Diego, CA. 1990). PCR reagents and protocols are also available from commercial vendors, such as Roche Molecular Systems.

The nucleic acids detected can be DNA or RNA molecules. In particular embodiments of the invention, RNA molecules are detected. The detected RNA molecules can also be RNA transcribed from genomic sequences, but which do not encode functional polypeptides. The first step in the amplification is the synthesis of a DNA copy (cDNA) of the region to be amplified. Reverse transcription can be carried out as a separate step, or in a homogeneous reverse transcription-polymerase chain reaction (RT-PCR), a modification of the polymerase chain reaction for amplifying RNA. Methods suitable for PCR amplification of ribonucleic acids are described in Romero and Rotbart in Diagnostic Molecular Biology: Principles and Applications pp.401-406, Persing et al. eds., (Mayo Foundation, Rochester, MN 1993); Rotbart et al. U.S. Patent No. 5,075,212 and Egger et al., J. Clin. Microbiol. 33:1442-1447 (1995)).

The primers used in the methods of the invention are preferably at least about 15 nucleotides to about 50 nucleotides in length, more preferably from about 15 nucleotides to about 30 nucleotides in length.

To amplify a target nucleic acid sequence in a sample by PCR, the sequence must be accessible to the components of the amplification system. In general, this accessibility is ensured by isolating the nucleic acids from the sample. A variety of techniques for extracting nucleic acids, from biological samples are known in the art and described above.

The first step of each cycle of the PCR involves the separation of the nucleic acid duplex formed by the primer extension. Once the strands are separated, the next step in PCR involves hybridizing the separated strands with primers that flank the target sequence. The primers are then extended to form complementary copies of the target strands. For successful PCR amplification, the primers are designed so that the position at which each primer hybridizes along a duplex sequence is such that an extension product synthesized from one primer, when separated from the template (complement), serves as a template for the extension of the other primer. The cycle of denaturation, hybridization, and extension is repeated as many times as necessary to obtain the desired amount of amplified nucleic acid (amplicon).

In the preferred embodiment of the PCR process, strand separation is achieved by heating the reaction to a sufficiently high temperature (∼95°C) for a sufficient time to cause the denaturation of the duplex but not to cause an irreversible denaturation of the polymerase (see U.S. Patent No. 4,965,188). Template-dependent extension of primers in PCR is catalyzed by a polymerizing agent in the presence of adequate amounts of four deoxyribonucleoside triphosphates (typically dATP, dGTP, dCTP, and dTTP) in a reaction medium comprised of the appropriate salts, metal cations, and pH buffering system. Suitable polymerizing agents are enzymes known to catalyze template-dependent DNA synthesis. In the present invention, the initial template for primer extension is typically first strand cDNA that has been transcribed from RNA. Reverse transcriptases (RTs) suitable for synthesizing a cDNA from the RNA template are well known.

PCR is most usually carried out as an automated process with a thermostable enzyme. In this process, the temperature of the reaction mixture is cycled through a denaturing region, a primer annealing region, and an extension reaction region automatically.

The nucleic acids can also be detected using other standard techniques, well known to those of skill in the art. Although the detection step is typically preceded by an amplification step, amplification is not required in the methods of the invention. For instance, the nucleic acids can be identified by size fractionation (*e*.*g*., gel electrophoresis). The presence of different or additional bands in the sample as compared to the control is an indication of the presence of target nucleic acids of the invention. Alternatively, the target nucleic acids can be identified by sequencing according to well known techniques. Alternatively, oligonucleotide probes specific to the target nucleic acids can be used to detect the presence of specific fragments.

As explained in detail below, the size of the amplified fragments produced by the methods of the invention is typically sufficient to identify the presence of one or more bands associated with a particular disease. Thus, in some embodiments of the invention, size fractionation (*e*.*g*., gel electrophoresis) of the amplified fragments produced in a given sample can be used to distinguish the fragments associated with a particular disease. This is typically carried out by amplifying a control with the same primers used to amplify the sample of interest. After running the amplified sequences out in an agarose or polyacrylamide gel and staining, the nucleic acid, *e*.*g*., with ethidium bromide or other stains such as fluorescence dyes, *e*.*g*., SYBR green™ (Molecular Probes) according to well known techniques (*see*, Sambrook *et al*.), the pattern of bands in the sample and control are compared. The presence of different or additional bands in the sample as compared to the control, is an indication of the presence of a band associated with a disease.

Sequence-specific probe hybridization is a well known method of detecting desired nucleic acids in a sample comprising cells, biological fluids and the like. Under sufficiently stringent hybridization conditions, the probes hybridize specifically only to substantially complementary sequences. The stringency of the hybridization conditions can be relaxed to tolerate varying amounts of sequence mismatch. If the target is first amplified, detection of the amplified product utilizes this sequence-specific hybridization to insure detection of only the correct amplified target, thereby decreasing the chance of a false positive.

A number of hybridization formats are well known in the art, including but not limited to, solution phase, solid phase, oligonucleotide array formats, mixed phase, or *in situ* hybridization assays. In solution (or liquid) phase hybridizations, both the target nucleic acid and the probe or primers are free to interact in the reaction mixture. Techniques such as real-time PCR systems have also been developed that permit analysis, *e*.*g*., quantification, of amplified products during a PCR reaction. In this type of reaction, hybridization with a specific oligonucleotide probe occurs during the amplification program to identify the presence of a target nucleic acid. Hybridization of oligonucleotide probes ensure the highest specificity due to thermodynamically controlled two state transition. Examples for this assay formats are fluorescence resonance energy transfer hybridization probes, molecular beacons, molecular scorpions, and exonuclease hybridization probes (reviewed in Bustin SM. J. Mol. Endocrin. 25:169-93 (2000)).

In solid phase hybridization assays, either the target or probes are linked to a solid support where they are available for hybridization with complementary nucleic acids in solution. Exemplary solid phase formats include Southern or Northern hybridizations, dot blots, arrays, chips, and the like. *In situ* techniques are particularly useful for detecting target nucleic acids in chromosomal material (*e*.*g*., in metaphase or interphase cells). The following articles provide an overview of the various hybridization assay formats: Singer et al., Biotechniques 4:230 (1986); Haase et al., METHODS IN VIROLOGY, Vol. VII, pp. 189-226 (1984); Wilkinson, IN SITU HYBRIDIZATION, D.G. Wilkinson ed., IRL Press, Oxford University Press, Oxford; and NUCLEIC ACID HYBRIDIZATION: A PRACTICAL APPROACH, Hames, B.D. and Higgins, S.J., eds., IRL Press (1987).

The hybridization complexes are detected according to well known techniques and are not a critical aspect of the present invention. Nucleic acid probes capable of specifically hybridizing to a target can be labeled by any one of several methods typically used to detect the presence of hybridized nucleic acids. One common method of detection is the use of autoradiography using probes labeled with ³H, 1²⁵I, ³⁵S, ¹⁴C, or ³²P, or the like. The choice of radioactive isotope depends on research preferences due to ease of synthesis, stability, and half-lives of the selected isotopes. Other labels include compounds (*e*.*g*., biotin and digoxigenin), which bind to antiligands or antibodies labeled with fluorophores, chemiluminescent agents, and enzymes. Alternatively, probes can be conjugated directly with labels such as fluorophores, chemiluminescent agents or enzymes. The choice of label depends on sensitivity required, ease of conjugation with the probe, stability requirements, and available instrumentation.

The probes and primers can be synthesized and labeled using well-known techniques. Oligonucleotides for use as probes and primers may be chemically synthesized according to the solid phase phosphoramidite triester method first described by Beaucage, S.L. and Caruthers, M.H., 1981, Tetrahedron Letts., 22(20):1859-1862 using an automated synthesizer, as described in Needham-VanDevanter, D.R., et al. 1984, Nucleic Acids Res., 12:6159-6168. Purification of oligonucleotides can be performed, *e*.*g*., by either native acrylamide gel electrophoresis or by anion-exchange HPLC as described in Pearson, J.D. and Regnier, F.E., 1983, J. Chrom., 255:137-149.

Detection of the nucleic acid sequences can also be accomplished by means of signal amplification techniques. For example, the branched DNA assay uses a specific probe to a target sequence to identify the presence of the target. The signal is amplified by means of modifications made to the probe which allow many fluorescent detector DNA molecules to hybridize to a target nucleic acid (Chiron Diagnostics).

Any nucleic acid species that exists in a particle-associated and non-particle associated form can be detected and used as a marker in the methods of the present invention. In a preferred embodiment, the markers human glyceraldehyde-3-phosphate dehydrogenase (GAPDH) mRNA, platelet basic protein (PBP) mRNA, or mitochondrial DNA are used to further evaluate disease conditions. Probes and primers of the invention for the detection of GAPDH mRNA, PBP mRNA or mitochondrial DNA can be synthesized using well-known techniques and are well known in the art (TaqMan probe and amplification primers from Perkin Elmer).

### Evaluating the disease condition

After separation of blood plasma or serum nucleic acids into two or more fractions such that the concentrations of particle-associated and non-particle associated nucleic acids in each fraction are different and following the detection and quantification of nucleic acids in each fraction by known methods, the disease condition is evaluated.

The disease condition is evaluated by determining the amount or concentration of nucleic acid in one or more fractions and comparing it to a control. The skilled practitioner can use the comparison to evaluate a disease condition in the patient. For example, when one fraction is processed by ultracentrifugation, in which case the supernatant will contain only non-particle-associated nucleic acids, a different concentration of non-particle associated nucleic acid in the sample than in the control processed in the same way indicates an abnormality in the patient. The greater the degree of difference between the concentration of non-particle associated nucleic acid in the patient and in the control, the greater the abnormality. By comparing the relative differences of non-particle associated nucleic acid in a patient and in a control, a skilled practitioner can determine if an individual is at risk for developing the disease. By comparing the relative differences of non-particle associated nucleic acid in a patient and in a control, a skilled practitioner can diagnose the disease, determine the stage of disease, or determine prognosis of the disease in the patient.

For example, in some patients, the concentration of non-particle associated nucleic acid circulating in his or her blood plasma or serum will be the same as in a control. In other patients, the amount or concentration of non-particle associated nucleic acid in the sample will be slightly higher than in the control, e.g., 50% higher. In even other patients, the amount or concentration of non-particle associated nucleic acid in the sample will be double that of the control. In yet even other patients, the amount or concentration of non-particle associated nucleic acid in the sample will be much higher than in the control, e.g., 8 to 15 fold higher. Depending upon the relative difference between the concentration of non-particle associated nucleic acid in the sample and in the control, a patient may be subject to different lengths of hospital stay or different treatment or monitoring plans. For example if the concentration of non-particle associated nucleic acid in the sample is equal to that in a control, a patient may be given a good prognosis and discharged from a hospital. In another example, if the concentration of non-particle associated nucleic acid in the sample is only slightly higher than in the control, a patient may be discharged from a hospital but subject to further monitoring. If the concentration of non-particle associated nucleic acid in the sample is much higher than that in the control, e.g., 15 to 100 fold higher, a patient may be given a poor prognosis and subject to more aggressive treatment.

In one aspect of the present invention, increased concentration of non-particle associated nucleic acids circulating in the blood plasma or serum of a patient can be used to evaluate cancer in a patient. Blood samples are obtained from patients suspected of having, known to have or at risk of having cancer. The plasma or serum sample is separated into two or more fractions using the methods of the present invention wherein each fraction contains a different relative concentration of particle-associated nucleic acids from the other fraction(s). Nucleic acids present in these fractions are extracted and detected using known methods. The concentrations of nucleic acids corresponding to each of these fraction are compared to a control. In a preferred embodiment, the *GAPDH* mRNA marker may be used to evaluate disease. Depending on the relative difference of nucleic acid in the sample from the patient and in the control, cancer can be diagnosed and further evaluated. In some embodiments, nasopharyngeal carcinoma can be diagnosed and further evaluated. In other embodiments, hepatocellular carcinoma can be diagnosed and further evaluated.

Separation of the nucleic acids in two or more fractions is not essential for the further evaluation of a patient having nasopharyngeal carcinoma or hepatocellular carcinoma. By comparing the concentration of total *GAPDH* mRNA in the plasma or serum sample from a patient having hepatocellular cancer or nasopharyngeal carcinoma to the concentration of total *GAPDH* mRNA in the plasma or serum sample of a control, the cancer can be detected and further evaluated. For example, a greater concentration of *GAPDH* mRNA in the sample than in the control indicates cancer in the patient.

In another aspect of the present invention, abnormal relative concentrations of particle-associated and non-particle associated nucleic acids circulating in the blood plasma or serum of a pregnant woman can be used to evaluate a disease or physiologic condition in the patient or her fetus. Blood samples are obtained from pregnant women and separated into fractions. These fractions are separated whereby the fractions will contain different relative concentrations of particle-associated and non-particle-associated nucleic acids. Nucleic acids from these fractions are then extracted and detected and measured using known methods. The concentrations of nucleic acids in these fractions are compared with each other and with a control. In some embodiments, a preferred marker is *GAPDH* mRNA. Depending on the relative difference between the nucleic acid concentrations between these fractions or in comparison with the control, a disease or physiologic condition in the patient or her fetus can be diagnosed or further evaluated.

Increased concentrations of non-particle associated nucleic acids circulating in the blood plasma or serum of a patient can be used to evaluate trauma in the patient. Blood samples are obtained from trauma patients. Plasma or serum is isolated and separated into two or more fractions with different relative concentrations of particle-associated and non-particle-associated nucleic acids. Nucleic acids are extracted from these fractions and detected and measured using known methods. Nucleic acid concentrations in these fractions are compared to a control. Preferred markers used to evaluate disease are *GAPDH* mRNA or mitochondrial DNA. Depending on the relative differences in nucleic acid concentrations in these fractions from the sample from a trauma patient and in the control, trauma can be further evaluated. For example, the severity of the trauma can be determined by comparing the concentration of nucleic acids in a fraction with predominantly non-particle associated nucleic acid from the trauma patient to a control. In some patients, a 4 to 6 fold increase in the concentration of non-particle associated nucleic acid in their blood plasma or serum as compared to a control will indicate that the patient is suffering from minor trauma. In other patients, a 7 to 10 fold increase will indicate that the patient is suffering from moderate trauma and and in other patients, an increase of 11 to 15 fold will indicate that the patient is suffering from major trauma.

### Informatics

In general, bioinformatics is the study and application of computer and statistical techniques to the management of biological information. The development of systems and methods to create and search databases containing biological information including concentrations of particle associated and non-particle associated circulating nucleic acids in the blood plasma or serum of healthy and diseased individuals and the ability to use that biological information to evaluate disease conditions is increasingly important.

A method for populating a database for further medical characterizaton is foreseen. The method includes populating a database with the blood plasma or serum particle associated and non-particle associated nucleic acid concentrations of patients suspected of having, known to have, or at risk of having specific diseases and using a software program to compare those concentrations to controls. A method for creating the control by amassing data from healthy and diseased individuals and corresponding concentrations of particle associated and non-particle associated nucleic acid concentration in their blood plasma or serum to absence or presence of disease, severity of disease, prognosis of disease, appropriate treatment plans for disease, or risk stratification, is foreseen.

Likewise an apparatus for automating the methods of the present invention, the apparatus comprising a computer and a software system capable of comparing inputed blood plasma or serum nucleic acid concentrations of patients suspected of having, known to have, or at risk of having specific diseases with controls. The nucleic acid concentration data is inputted in computer-readable form and stored in computer-retrievable form. The computer-readable medium may be encoded with a data set comprising concentration of particle associated and non-particle associated blood plasma or serum nucleic acid levels in patients suspected of having, known to have or at risk of having specific diseases.

The methods described herein for quantifying particle associated and non-particle associated nucleic acid concentrations in the blood plasma or serum of individuals provide information which can be correlated with pathological conditions, predisposition to disease, therapeutic monitoring, prognosis, risk stratification, among others. Although the data generated from the methods of the invention is suited for manual review and analysis, in a preferred embodiment, prior data processing using high-speed computers is utilized.

An array of methods for indexing and retrieving biomolecular information is known in the art. For example, U.S. Patents 6,023,659 and 5,996,712 disclose a relational database system for storing biomolecular sequence information in a matter that allows sequences to be catalogued and searched according to one or more protein function hierarchies.

Storage and retrieval of a collection of blood plasma or serum nucleic acid concentrations in a computer data storage apparatus, which can include magnetic disks, optical disks, magneto-optical disks, DRAM, SRAM, SGRAM, SDRAM, RDRAM, DDR RAM, magnetic bubble memory devices, and other data storage devices, including CPU registers and on-CPU data storage arrays, is foreseen.

Likewise a magnetic disk, such as an IBM-compatible (DOS, Windows, Windows95/98/2000, Windows NT, OS/2) or other format (*e*.*g*., Linux, SunOS, Solaris, AIX, SCO Unix, VMS, MV, Macintosh, *etc*.) floppy diskette or hard (fixed, Winchester) disk drive, comprising a bit pattern encoding data collected from the methods of the present invention in a file format suitable for retrieval and processing in a computerized sequence analysis, comparison, or relative quantitation method, is disclosed.

A network, comprising a plurality of computing devices linked via a data link, such as an Ethernet cable (coax or 10BaseT), telephone line, ISDN line, wireless network, optical fiber, or other suitable signal tranmission medium, whereby at least one network device (*e*.*g*., computer, disk array, *etc*.) comprises a pattern of magnetic domains (*e*.*g*., magnetic disk) and/or charge domains (*e*.*g*., an array of DRAM cells) composing a bit pattern encoding data acquired from the methods of the invention, is disclosed, as well as a method for transmitting concentration data that includes generating an electronic signal on an electronic communications device, such as a modem, ISDN terminal adapter, DSL, cable modem, ATM switch, or the like, wherein the signal includes (in native or encrypted format) a bit pattern encoding data collected from the methods of the present invention.

A computer system for comparing particle associated and non-particle associated nucleic acid concentration in blood plasma or serum of disease individuals to a control is disclosed. A central processor is preferably initialized to load and execute the computer program for alignment and/or comparison of the assay results. Data is entered into the central processor via an I/O device. Execution of the computer program results in the central processor retrieving the data from the data file.

The target data or record and the computer program can be transferred to secondary memory, which is typically random access memory (*e*.*g*., DRAM, SRAM, SGRAM, or SDRAM). For example, a central processor can be a conventional computer (*e*.*g*., Intel Pentium, PowerPC, Alpha, PA-8000, SPARC, MIPS 4400, MIPS 10000, VAX, *etc*.); a program can be a commercial or public domain molecular biology software package (*e*.*g*., UWGCG Sequence Analysis Software, Darwin); a data file can be an optical or magnetic disk, a data server, a memory device (*e*.*g*., DRAM, SRAM, SGRAM, SDRAM, EPROM, bubble memory, flash memory, *etc*.); an I/O device can be a terminal comprising a video display and a keyboard, a modem, an ISDN terminal adapter, an Ethernet port, a punched card reader, a magnetic strip reader, or other suitable I/O device.

The use of a computer system, such as that described above, which comprises: (1) a computer; (2) a stored bit pattern encoding a collection of nucleic acid concentrations obtained by the methods of the invention, which may be stored in the computer; (3) a comparison control (4) a program for comparison is foreseen. It is understood that the examples and embodiments described herein are for illustrative purposes only.

### EXAMPLES

### Example 1:

### Detection of particle and non-particle associated circulating nucleic acids in the plasma or serum of healthy patients

Plasma samples from 17 healthy subjects were analyzed for human *glyceraldehyde-3-phosphate dehydrogenase* (*GAPDH*) mRNA and human *beta-globin* DNA concentrations. Amongst the 17 plasma samples, 14 of them were further analyzed for human mitochondrial DNA concentrations. Serum samples from 10 out of the 17 healthy subjects were analyzed for *GAPDH* mRNA and *beta-globin* DNA concentrations. Aliquots from each sample were individually passed through filters with 5 µm, 0.45 µm and 0.22 µm pore sizes prior to quantitative analysis. Data in Fig. 1A show that filtration had a clearly observable effect on *GAPDH* mRNA concentrations in plasma and serum samples (Friedman test, P < 0.001 for both plasma and serum samples). Pairwise analysis further indicates that statistically significant difference is detected in every pair of these filter sizes (Student-Newman-Keuls test, P < 0.05 for each pair). Overall, plasma and serum *GAPDH* mRNA concentration decreased by a median of 15-fold (interquartile range: 10-fold to 24-fold) and 8.7-fold (interquartile range: 6.6-fold to 11.5-fold), respectively, when comparing paired samples from the unfiltered and 0.22 µm filtered groups. Thus, our data clearly indicate that filterable *GAPDH* mRNA species are present in the plasma and serum of human subjects. Data in Fig. 1B show that similar filtration effect was obtained for the mitochondrial DNA concentrations in the plasma samples. Significant difference in mitochondrial DNA concentrations was found among different pore-sized filtered plasma samples (Friedman test, P < 0.001). Pairwise analysis shows that the significant difference was detected for all but one of the paired comparisons (Student-Newman-Keuls test, P < 0.05). There was no significant difference between the unfiltered plasma and plasma filtered through 5 µm filter (Student-Newman-Keuls test, P > 0.05). Overall, plasma mitochondrial DNA concentration decreased by a median of 8.9-fold (interquartile range: 2.8-fold to 23.7-fold) when comparing paired samples from the unfiltered and 0.22 µm filtered groups. In contrast to these results, there is no statistically significant difference in *beta-globin* DNA concentrations among different pore-sized filtered plasma and serum samples (Friedman test, *P* = 0.455 for plasma samples; and *P* = 0.516 for serum samples) (Fig. 1C). Apart from investigating the particle-associated nature of *GAPDH* mRNA, we also analyzed a human gene transcript named *platelet basic protein* (*PBP*). Plasma and serum samples from 8 out of the 17 healthy subjects were used in this analysis. Fig. 1D shows that filtration effect on *PBP* mRNA concentrations was once again clearly observed. Overall, plasma and serum *PBP* mRNA concentration decreased by a median of 160-fold (interquartile range: 41-fold to 427-fold) and 38-fold (interquartile range: 15.7-fold to 52-fold), respectively, when comparing paired samples from the unfiltered and 0.22-µm filtered groups. These results therefore indicate that a significant proportion of *GAPDH* mRNA, *PBP* mRNA and mitochondrial DNA in plasma and serum are particle-associated. On the other hand, most of the circulating *beta-globin* DNA is non-particle-associated.

To investigate if any nucleic acids in plasma existed in a *non*-particle-associated form, we performed a second series of experiments using ultracentrifugation. Plasma samples from another 10 healthy subjects were analyzed *GAPDH* mRNA and *beta-globin* DNA concentrations. Amongst the 10 plasma samples, 7 of them were further analyzed for mitochondrial DNA concentration. Aliquots from each sample were subjected to either a 0.22-µm filtration step or ultracentrifugation at 99,960 X g prior to quantitative analysis. In a previous study, Yamamoto et al have used centrifugal forces of 70,000 X g to pellet viral particles (Yamamoto M et al, J Clin Microbiol 1995;33:1756-8). The even more intensive centrifugal force (99,960 X g) used in our study would therefore be expected to pellet virtually all particulate matter. Data in Fig. 2A and 2B show that statistically significant differences are present in both *GAPDH* mRNA and mitochondrial DNA concentrations in unfiltered plasma, 0.22-µm filtered plasma and ultracentrifuged plasma (Friedman test, *P* < 0.001 for both *GAPDH* mRNA and mitochondrial DNA). Pairwise comparisons indicate significant difference exists between unfiltered plasma and 0.22-µm filtered plasma (Student-Newman-Keuls Test, *P* < 0.05 for both *GAPDH* mRNA and mitochondrial DNA), and between unfiltered plasma and ultracentrifuged plasma (Student-Newman-Keuls Test, *P* < 0.05 for both *GAPDH* mRNA and mitochondrial DNA). However, there is no significant difference in both *GAPDH* mRNA and mitochondrial DNA concentrations between the 0.22-µm filtered plasma samples and the ultracentrifuged plasma samples (Student-Newman-Keuls test, *P* > 0.05 for both *GAPDH* mMA and mitochondrial DNA) (Fig. 2A and 2B). On the other hand, the differences in *beta-globin* DNA concentrations among different treated plasma samples do not reach any statistical significance (Friedman test, *P* = 0.122) (Fig. 2C). It is interesting to note, therefore, that even following this ultracentrifugation step, a certain amount of *GAPDH* mRNA and mitochondrial DNA was still detectable in the supernatant. These signals represented a median of 7.4% (interquartile range: 4.3% to 13%) and 15.4% (interquartile range: 5.3% to 39.3%) of the original *GAPDH* mRNA and mitochondrial DNA concentrations, respectively, that were present in the non-ultracentrifuged plasma samples and were likely to represent non-particle-associated circulating nucleic acid species.

### Example 2

### Existence of particle- and non-particle-associated nature of circulating nucleic acids in the plasma of clinical conditions -Hepatocellular carcinoma (HCC)

Plasma samples from 16 HCC patients were analyzed. Fig. 3A shows that particle-associated *GAPDH* mRNA was present in the plasma of HCC patients. Indeed, filtration with a 0.22-µm filter resulted in a median of 9.3-fold (interquartile range: 6.9-fold to 31.1-fold) reduction in *GAPDH* mRNA concentration in plasma. In 7 out of the 16 HCC cases, enough plasma samples had been collected for an additional experiment involving plasma *beta-globin* DNA quantitation. No statistically significant difference is observed for plasma *beta-globin* DNA concentrations between HCC patients and healthy subjects, both with or without filtration (Mann-Whitney Rank Sum test, *P* = 0.525 for filtered plasma samples; and *P* = 0.418 for unfiltered plasma samples) (Fig. 3B). Thus, filtration with a 0.22-µm filter resulted in a marked reduction in plasma *GAPDH* mRNA concentrations in HCC patients (Fig. 3A). Interestingly, we have also found that with or without filtration, *GAPDH* mRNA concentrations in the plasma of HCC patients were significantly higher than those in healthy subjects (Mann-Whitney Rank Sum test, *P* < 0.05 for filtered plasma samples; and *P* < 0.005 for unfiltered plasma samples) (Fig. 3A). These results suggest that the plasma of HCC patients contains increased concentrations of both particle-associated and non-particle-associated mRNA species. With regard to the particle-associated mRNA species, one possibility is that apoptosis of neoplastic cells in cancer patients might release apoptotic bodies packaged with RNA into plasma.

### Example 3

### Existence of particle- and non-particle-associated nature of circulating nucleic acids in the plasma of clinical conditions - Nasopharyngeal carcinoma (NPC)

To see if particle-associated nucleic acids also exist in other cancers, plasma samples from 7 NPC patients were analyzed for *GAPDH* mRNA and *beta-globin* DNA concentrations. Data in Fig. 4A show that particle-associated *GAPDH* mRNA was present in the plasma of NPC patients. Filtration with a 0.22-µm filter resulted in a median of 3.3-fold (interquartile range: 3-fold to 7.5-fold) reduction in *GAPDH* mRNA concentration in plasma. Similar to HCC patients, plasma *GAPDH* mRNA concentrations in NPC patients were significantly higher than those in healthy subjects, both with or without filtration. (Mann-Whitney Rank Sum test, *P* < 0.05 for both filtered and unfiltered plasma samples) (Fig. 4A). In contrast, no statistically significant difference is observed for plasma *beta-globin* DNA concentrations between NPC patients and healthy subjects, both with or without filtration (Mann-Whitney Rank Sum test, *P* = 0.065 for filtered plasma samples; and *P* = 0.155 for unfiltered plasma samples) (Fig. 4B).

### Example 4

### Existence of particle- and non-particle-associated nature of circulating nucleic acids in the plasma of clinical conditions - Pregnancy

Plasma samples from 15 pregnant women of 16 weeks to 20 weeks of gestation were analyzed for *GAPDH* mRNA and *beta-globin* DNA concentrations. Data in Fig. 5A clearly show that particle-associated *GAPDH* mRNA was also present in the plasma of pregnant women. A 0.22-µm pore-sized filtration step resulted in a median of 16.6-fold (interquartile range: 14-fold to 28.3-fold) reduction in *GAPDH* mRNA concentration in plasma. Unlike HCC and NPC patients, plasma *GAPDH* mRNA concentrations in pregnant women were significantly lower than those in healthy subjects, both with or without filtration. (Mann-Whitney Rank Sum test, *P* < 0.001 for both filtered and unfiltered plasma samples) (Fig. 5A). Similarly, no statistically significant difference is observed for plasma *beta-globin* DNA concentrations between pregnant women and healthy subjects, both with or without filtration (Mann-Whitney Rank Sum test, *P* = 0.265 for filtered plasma samples; and *P* = 0.628 for unfiltered plasma samples) (Fig. 5B).

To determine if particle-associated RNA exists throughout the whole period of pregnancy, plasma samples from 10, 22 and 4 pregnant women of first, second and third trimesters respectively were analyzed for *GAPDH* mRNA. Data in Fig. 5C show that particle-associated *GAPDH* mRNA was present in maternal plasma during pregnancy of all stages. For all stages of gestation, significant reduction in *GAPDH* mRNA concentrations were observed when plasma samples were subjected to 0.22-µm filtration treatments (Mann-Whitney Rank Sum test, P < 0.001 for first, second and third trimesters).

Apart from *GAPDH* mRNA, two placental mRNA species, *human placental lactogen (hPL)* mRNA and the *beta-subunit of human chorionic gonadotropin (βhCG)* mRNA, were investigated for their particle-associated nature in maternal plasma. Plasma samples from 14 pregnant women with gestation from 7 weeks to 14 weeks were collected. Aliquots from each sample were individually passed through filters with 5 µm and 0.45 µm pore sizes or remained unfiltered. They are then subjected to quantitative analysis for *hPL, bhCG and GAPDH* mRNA. Data in Fig. 6 show a significant effect of filtration on plasma *hPL* (Fig. 6A) and β*hCG* (Fig. 6B) mRNA concentrations (Friedman test, P < 0.001 for both *hPL* and *βhCG* mRNA). Pairwise analysis shows that statistically significant difference is detected between the 5 µm and 0.45 µm filtered groups (Dunn's test, P < 0.05 for both *hPL* and *βhCG* mRNA). Overall, plasma *hPL* and *βhCG* mRNA levels decreased by median values of 3.9-fold (interquartile range: 1.8-fold to 5.5-fold) and 3.5-fold (interquartile range: 2.8-fold to 9.0-fold), respectively, when comparing paired samples from the unfiltered and 0.45-µm filtered groups. Fig 6C shows the *GAPDH* mRNA concentrations of the corresponding maternal plasma samples. As expected, significant difference was detected among different treatments groups (Friedman test, P < 0.001). Pairwise analysis showed that significant difference were detected between the 5-µm and 0.45-µm filtered groups (Dunn's test, P < 0.05). These data therefore demonstrates that a significant proportion of placental derived mRNA, i.e., *hPL* and *βhCG* mRNA, are associated with subcellular particulate matter in maternal plasma.

### Example 5

### Existence of particle- and non-particle-associated nature of circulating nucleic acids in the plasma of clinical conditions - Trauma - GAPDH

Blood samples were collected from patients who had traumatic injury requiring admission to the Emergency Resuscitation Room at the Prince of Wales Hospital. Ethics approval was obtained from the Research Ethics Committee of the Chinese University of Hong Kong. The total extent of anatomical injury, as determined in the emergency department, was calculated objectively using the Injury Severity Score (ISS) (Baker SP et al. J. Trauma 1974;14:187-196). According to the ISS, trauma patients were divided into three groups: minor injury (ISS < 9), moderate injury (ISS = 9 to 15) and severe injury (ISS > 15). The injury severity scores took into account only injuries detected in the emergency room and did not include computed axial tomography findings.

Five to ten ml of blood samples were collected into EDTA-tubes. The samples were centrifuged at 1500 g at 4 degrees Celsius, and plasma was carefully removed from EDTA-containing tubes, and transferred into plain polypropylene tubes. Great care was taken to ensure that the buffy coat was undisturbed when plasma samples were removed. Each plasma sample was divided into two fractions: one-half was filtered with a 0.22 µm filter and the remaining fraction remained unfiltered.

RNA from the plasma samples were extracted using a RNeasy Mini Kit (Qiagen, Hilden, Germany). 600 to 1000 µl of plasma was mixed with 1.2 ml of Trizol LS reagent (Invitrogen) and 0.2 ml of chloroform. The mixture was centrifuged at 11,900 g for 15 minutes and the aqueous layer was transferred into new tubes. One volume of 70% ethanol was added to one volume of the aqueous layer. The mixture was then applied to the RNeasy mini column and was processed according to the manufacturer's recommendations. Total RNA was eluted with 15 µl of RNase-free water followed by DNase I (Invitrogen) treatment. RNA was stored at -80 degrees Celsius until further processing.

Real time quantitative RT-PCR analysis was performed using a Applied Biosystems 7700 Sequence Detector (Foster City, Calif., U.S.A.), which is essentially a combined thermal cycler/fluorescence detector with the ability to monitor the progress of individual PCR reactions optically. The amplification and product reporting system used is based on the 5' nuclease assay (Holland P. M. et al., Proc. Natl. Acad. Sci. USA 1991;88:7276-7280)(the TaqMan assay as marketed by Perkin-Elmer). In this system, apart from the two amplification primers as in conventional PCR, a dual labeled fluorogenic hybridisation probe is also included (Livak KJ et al. Nat. Genet. 1995;9:341-342). One fluorescent dye serves as a reporter (FAM, i.e., 6-carboxyfluorescein) and its emission spectrum is quenched by a second fluorescent dye (TAMRA, i.e., 6-carboxy-tetramethylrhodamine). During the extension phase of PCR, the 5' to 3'-exonuclease activity of the Taq DNA polymerase cleaves the reporter from the probe thus releasing it from the quencher, resulting in an increase in fluorescent emission at 518 nm. The Applied Biosystems 7700 Sequence Detector is able to measure the fluorescent spectra of the 96 amplification wells continuously during DNA amplification and the data are captured onto a Macintosh computer (Apple Computer, Cupertino, Calif., U.S.A.).

One-step real-time quantitative RT-PCR system was used for the measurement of mRNA concentration in plasma. In this system, the *rTth* DNA polymerase functioned both as a reverse transcriptase and a DNA polymerase. The *GAPDH* TaqMan system consisted of the amplification primers GAPDH-F, 5'-GAA GGT GAA GGT CGG AGT-3'; GAPDH-R, 5'-GAA GAT GGT GAT GGG ATT TC-3'; and the dual-labeled fluorescent TaqMan probe was GAPDH-P, 5'-(FAM)CAA GCT TCC CGT TCT CAG CC(TAMRA)-3'. Sequence data for the human *GAPDH* gene were obtained from the GenBank Sequence Database (accession number M33197).

RT-PCR was set up in a reaction volume of 50 µl using components (except TaqMan probe and amplification primers) supplied in an EZ r*Tth* RNA PCR kit and a TaqMan^{®} *GAPDH* Control Reagents (Applied Biosystems). The *GAPDH* TaqMan probe was custom-synthesized by Applied Biosystems. PCR primers were synthesized by Genset Oligo (Singapore). Each reaction contained 10 µl of 5X EZ buffer; 200 nM of each primer; 100 nM of the fluorescent probe; 3 mM Mn(OAc)₂; 300 µM each of dATP, dCTP, dGTP; 600 µM dUTP; 5 units of r*Tth* polymerase; and 0.5 unit of AmpErase uracil N-glycosylase. 3 µl of extracted RNA was used for amplification. The exact amount used was recorded for subsequent concentration calculation. Strict precautions against RT-PCR contamination were used. Aerosol-resistant pipette tips were used for all liquid handling. Separate areas were used for the setting up of amplification reactions, the addition of RNA template and the carrying out of amplification reactions. The 7700 Sequence Detector offered an extra level of protection that its optical detection system obviated the need to reopen the reaction tubes following the completion of the amplification reactions, thus minimizing the possibility of carryover contamination. In addition, the TaqMan assay also included a further level of anticontamination measure in the form of pre-amplification treatment using uracil N-glycosylase which destroyed uracil. Amplifications were carried out in 96-well reaction plates that were frosted by the manufacturer to prevent light reflection and were closed using caps designed to prevent light scattering. Each sample was analyzed in duplicate and multiple negative water blanks were included in every analysis. A calibration curve for *GAPDH* quantification was prepared by subjecting serial dilutions of human control RNA (Applied Biosystems), with RNA concentrations ranging from 15 ng to 0.23 pg, to the RT-PCR assay. The manufacturer estimated that 1 pg of this RNA standard contained approximately 100 copies of *GAPDH* transcript.

The thermal profile used for the *GAPDH* system was as follows: before reverse transcription, reaction was initiated at 50°C for 2 minutes in the presence of uracil N-glycosylase, followed by a reverse transcription step at 60°C for 30 minutes. After a 5-minute denaturation at 95°C, PCR was carried out for 40 cycles using a denaturation step of 94°C for 20 seconds and an annealing/extension step of 60°C for 1 minute. Amplification data collected by the 7700 Sequence Detector and stored in the Macintosh computer were then analysed using the Sequence Detection System (SDS) software developed by Applied Biosystems. The mean quantity of each duplicate was used for further concentration calculation. The concentration expressed in pg/ml was calculated using the following equation: C = Q X V_{RNA} /V_{PCR}X 1/Vₑₓₜ, where C = target concentration in plasma (pg/ml); Q = target quantity (pg) determined by sequence detector in a RT-PCR; V_{RNA} = total volume of RNA obtained following extraction, typically 15 µl per Qiagen extraction; V_{PCR} = volume of RNA solution used for RT-PCR, typically 3-5 µl;Vₑₓₜ = volume of plasma extracted, typically 600-800 µl.

Statistical analysis was performed using the Sigma Stat 2.03 software (SPSS). containing PCR products.

To assess the linearity of the assay, serial dilutions of the control RNA were analyzed by the *GAPDH* RT-PCR system. The sensitivity of the RT-PCR system was sufficient to detect the *GAPDH* transcripts present in 0.23 pg of the control RNA (i.e. approximately 23 copies) (FIG. 7A). The calibration curve showed a correlation coefficient of 0.995 (Fig. 7B). To assess the precision of both the RNA extraction and the RT-PCR steps, 10 replicate extractions from a plasma sample obtained from a healthy individual were performed and these extracted RNA samples were subjected to real-time quantitative RT-PCR analysis. The coefficient of variation of Ct values of these replicate analyses was 1.66 %. A parameter, termed the threshold cycle (C_{T}) could be defined which was set at 10 standard deviations above the mean base-line fluorescence calculated from cycles 1 to 15 and was proportional to the starting target copy number used for amplification. A plot of the threshold cycle (C_{T}) against the input target quantity, with the latter plotted on a common log scale, demonstrated the large dynamic range and accuracy of real time quantitative RT-PCR.

The particle-associated nature of plasma RNA in trauma patients was investigated. Paired unfiltered and 0.22-µm filtered plasma samples from 8 trauma patients were analyzed for *GAPDH* mRNA concentrations. As shown in Fig. 8, particle-associated *GAPDH* mRNA was present in trauma patients. A 0.22-µm filtration step resulted in a median of 70.1-fold (interquartile range: 13.03-fold to 112.8-fold) reduction in *GAPDH* mRNA concentration in plasma. It is also found that the concentrations of *GAPDH* mRNA in the filtered plasma samples was significantly higher in trauma patients than control subjects (Mann-Whitney Rank Sum test, P < 0.05) whereas no significant difference in *GAPDH* mRNA concentrations was obtained between the unfiltered plasma samples of trauma and control groups (Mann-Whitney Rank Sum test, P = 0.962) (Fig. 8). There is a median of 9.9-fold increase in *GAPDH* mRNA concentration in the filtered plasma of trauma patients when compared to that of control groups. Our data indicate that non particle-associated *GAPDH* mRNA species are elevated in the plasma of trauma patients when compared with healthy control group. The elevation may due to the injured cells mainly released non particle-associated RNA after trauma.

Based on the Injury Severity Score (ISS), 17 trauma patients with available filtered plasma samples were categorized into severe injury (6 patients), moderate injury (5 patients), and minor injury (6 patients). Data in Fig. 9A show that the *GAPDH* mRNA concentrations in 0.22-µm filtered plasma were significantly different among normal controls and the trauma patients of different injury levels (Kruskal-Wallis test, P < 0.001). A positive correlation between the *GAPDH* mRNA concentrations and the injury severity was demonstrated. The median *GAPDH* mRNA concentration of the minor, moderate and major injury groups were increased by 6-, 17.4- and 20.6-fold respectively, when comparing with the normal controls. These data further indicates that the non particle-associated *GAPDH* mRNA species in plasma may be important for risk-stratification and disease monitoring in trauma patients.

Among the 6 trauma patients with major injury, 4 patients survived and 2 patients died eventually. 1 out of the 4 survived patients and 1 out of the 3 patients who later died had developed multiple organ dysfunction syndrome (MODS). Their filtered plasma *GAPDH* mRNA concentrations were shown in Fig 9B. It is interesting to note that although no significant difference was detected between the survived group and the groups that did not survive (Mann-Whitney Rank Sum test, P = 0.133), the median *GAPDH* mRNA concentration of the group that did not survive was increased by 5.9-fold when compared to that of the survived group.

### Example 6

### Existence of particle- and non-particle-associated nature of circulating nucleic acids in the plasma of clinical conditions - Trauma - mitochondrial DNA

It has now been demonstrated that circulating plasma DNA in the blood samples of trauma patients increases early after injury and these increases are related to the post-traumatic complications (Lo YMD et al. Clin. Chem. 2000;46:319-323). In the first hour after injury, levels of plasma DNA increase by 100-fold compared with healthy controls. A 10- to 18-fold increase in median levels is obtained in those who develop organ failure (OF), multiple organ dysfunction syndrome (MODS), and acute lung injury (ALI) and in those who die, compared with patients with uncomplicated injury. Despite having sensitivities and specificities of 74% to 100% at optimal cutoff points, circulating plasma DNA generated positive predictive values of 63% to 77% for OF and 33% to 40% for MODS (Rainer TH et al. Ann. NY Acad. Sci. 2001;945:211-220). By combining the predictive strengths of plasma DNA and other variables (e.g., aspartate transaminase) into a prediction guideline, the prediction models possibly offered overall correct classifications of 87% to 93% and positive predictive values of 85% for OF and 50% for MODS.

Since each cell contains hundreds to thousands of mitochondria and each mitochondrion has several copies of mitochondrial DNA, it has also been demonstrated that the release of mitochondrial DNA in the plasma of trauma patients is significantly higher than that of normal controls. With regard to the detection and quantification of mitochondrial DNA in trauma conditions, it will be useful to serve as a sensitive marker for prognostication, monitoring, evaluation, and risk stratification of trauma conditions.

Blood samples were collected from patients who had traumatic injury or stroke requiring admission to the Emergency Resuscitation Room at the Prince of Wales Hospital. Ethics approval was obtained from the Research Ethics Committee of the Chinese University of Hong Kong. The total extent of anatomical injury, as determined in the emergency department, was calculated objectively using the Injury Severity Score (ISS) (Baker SP et al. J. Trauma 1974;14:187-196). According to the ISS, trauma patients were divided into three groups: minor injury (ISS < 9), moderate injury (ISS = 9 to 15) and severe injury (ISS > 15). The injury severity scores took into account only injuries detected in the emergency room and did not include computed axial tomography findings.

Five to ten ml of blood samples were collected into EDTA-tubes. The samples were centrifuged at 1500 g for 10 minutes, and plasma were carefully removed from EDTA-containing tubes, and transferred into plain polypropylene tubes. Great care was taken to ensure that the buffy coat was not disturbed. Each plasma sample was filtered with a 0.22 µm filter. The samples were then stored at -20 until further processing.

DNA from plasma were extracted using a QIAamp Blood Kit (Qiagen, Hilden, Germany) using the "blood and body fluid protocol" as recommended by the manufacturer. 400 µl to 800 µl of plasma sample was used for DNA extraction per column.

The mitochondrial DNA TaqMan system consisted of the amplification primers Mit 3130F, 5'-AGG ACA AGA GAA ATA AGG CC-3', and Mit 3301R, 5'-TAA GAA GAG GAA TTG AAC CTC TGA CTG TAA-3', which were previously reported (Parfait et al., Biochem Biophys Res Commun 1998;247:57-59). The TaqMan probe sequence, Mit 3153T, 5'-FAM-TTC ACA AAG CGC CTT CCC CCG TAA ATG A-TAMRA-3' was designed using Primer Express software (Applied Biosystems). Sequence data for the mitochondrial DNA segment were obtained from the GenBank Sequence Database (accession number J01415). All components of the PCR other than the primers and probes were supplied in the TaqMan PCR Core Reagent Kit (Applied Biosystems). PCR was set up in a reaction volume of 50 µL according to the manufacturer's instructions. We added 5 µL of plasma DNA to each reaction mixture, which consisted of 5 µL of 10x buffer A; 300 nM each primer; 50 nM TaqMan probe; 4 mM MgCl₂; 200 nM each of dATP, dCTP, and dGTP; 400 nM dUTP; 1.25 U of AmpliTaq Gold; and 0.5 U of AmpErase uracil N-glycosylase. A calibration curve for mitochondrial DNA quantification was prepared by subjecting serial dilutions of the mitochondrial DNA amplicon that was subcloned in a plasmid vector (TOPO^{®} TA cloning reagent set, Invitrogen). The calibration curve was run in parallel and in duplicate with each analysis, with concentrations ranging from 100,000 copies to 1 copy of mitochondrial DNA. Real-time quantitative PCR was carried out in an Applied Biosystems 7700 Sequence Detector. The following thermal profile was used: incubation for 2 min at 50 °C, followed by a first denaturation step of 10 min at 95 °C and 45 cycles of 95 °C for 15 s and 55 °C for 1 min. Each sample was analyzed in duplicate. The mean quantity of each duplicate was used for further concentration calculation. The concentration expressed in copies/ml was calculated using the following equation: C = Q X V_{DNA} /V_{PCR} X 1/Vₑₓₜ, where C = target concentration in plasma (copies/ml); Q = target quantity (copies) determined by sequence detector in a PCR; V_{DNA} = total volume of DNA obtained following extraction, typically 50 µl per Qiagen extraction; V_{PCR} = volume of DNA solution used for PCR, typically 5 µl;Vₑₓₜ = volume of plasma extracted, typically 600-800 µl.

Statistical analysis was performed using the Sigma Stat 2.03 software (SPSS). To determine the dynamic range of real time quantitative PCR, serial dilutions of the cloned plasmid DNA representing mitochondrial DNA concentrations equivalent from 100,000 copies to 1 copy per reaction was subjected to analysis by the mitochondrial DNA TaqMan system. The assay was linear over five orders of magnitude (Fig. 10), and its sensitive allowed the detection of 1 copy of mitochondrial DNA.

Particle-associated nature of plasma mitochondrial DNA in trauma patients was investigated. Paired unfiltered and 0.22-µm filtered plasma samples from 37 trauma patients were analyzed for mitochondrial mRNA concentrations. Data in Fig. 11 show that particle-associated mitochondrial DNA was also present in the plasma of trauma patients. A 0.22-µm filtration step resulted in a median of 266.7-fold (interquartile range: 75.0-fold to 558.4-fold) reduction in mitochondrial DNA concentration in plasma. It is also found that concentrations of mitochondrial DNA in both the filtered the unfiltered plasma samples were significantly higher in trauma patients than control subjects (Mann-Whitney Rank Sum test, *P* < 0.05 for filtered and unfiltered plasma). There is a median of 6.3-fold and 5.3-fold increase in mitochondrial DNA concentration in the filtered and unfiltered plasma of trauma patients, respectively, when compared to that of control groups. Our data indicate that both filterable and non-filterable mitochondrial DNA species are elevated in the plasma of trauma patients when compared with healthy control group. These elevations may due to the injured cell released both particle- and non particle-associated mitochondrial DNA into the circulation after trauma.

Unfiltered plasma samples obtained from 27, 18 and 42 trauma patients of severe, moderate and minor injury respectively, as well as 0.22-µm filtered plasma samples obtained from 19, 12 and 24 trauma patients of severe, moderate and minor injury respectively. were analyzed for mitochondrial DNA concentrations. For both unfiltered and filtered plasma samples, the mitochondrial DNA concentrations were significantly different among the normal controls and the trauma patients of different injury levels (Kruskal-Wallis test, P < 0.001) (Fig. 12). For unfiltered plasma samples, the median mitochondrial DNA concentrations of the minor, moderate and major injury groups were increased by 9.9-, 6.4-, 6.4- and 11.4-fold respectively, when comparing with the normal controls (Fig. 12A). For filtered plasma samples, the median mitochondrial DNA concentrations of the minor, moderate and major injury groups were elevated by 2.5-, 6.4- and 9.9-fold respectively when compared to that of the normal controls (Fig. 12B).

## Claims

1. A method of evaluating a disease condition in a patient suspected of suffering or known to suffer from the disease condition, said method comprising:
(i) isolating plasma or serum from the blood sample obtained from the patient suspected of suffering or known to suffer from a disease condition,
(ii) separating the plasma or serum into two or more fractions such that particle associated nucleic acid and non-particle-associated nucleic acid are present in two different fractions; and
(iii) evaluating the disease condition by comparing the relative amount or concentration or characteristic of particle-associated and non-particle-associated nucleic acid of a nucleic acid species in said fractions and comparing the relative amount or concentration or characteristic of the nucleic acid to a control.

2. The method of claim 1, wherein the nucleic acid is a mRNA or a DNA.

3. The method of claim 2, wherein the mRNA is selected from the group of glyceraldehyde-3-phosphate dehydrogenase (GAPDH) mRNA, and platelet basic protein mRNA.

4. The method of any preceding claim, further comprising the step of amplifying the nucleic acid.

5. The method of claim 4, wherein the nucleic acid is amplified by PCR or reverse-transcriptase PCR.

6. The method of claim 5, wherein the nucleic acid is amplified by real-time PCR or real-time reverse-transcriptase PCR.

7. The method of any preceding claim wherein the fractions are separated by filtration.

8. The method of claim 7, wherein the filter size is about 5 µm or smaller.

9. The method of claim 8, wherein the filter size is about 0.22 µm.

10. The method of claim 8, wherein the filter size is about 0.45 µm.

11. The method of any one of claims 1 to 6, wherein the fractions are separated by centrifugation.

12. The method of claim 11, wherein the centrifugation is ultracentrifugation.

13. The method of any preceding claim wherein the disease condition is selected from the group consisting of cancer, stroke, and trauma.

14. The method of claim 13, wherein the cancer is hepatocellular cancer or nasopharyngeal carcinoma.

15. The method of any preceding claim wherein the patient is pregnant and the method of evaluating a disease or physiologic condition in the patient or her fetus aids in the detection, monitoring, prognosis or treatment of the patient or her fetus.

## Patentansprüche

1. Verfahren zum Evaluieren eines Erkrankungszustands in einem Patient von dem vermutet wird oder von dem bekannt ist, dass er an dem Erkrankungszustand leidet, wobei das Verfahren umfasst:
(i) Isolieren von Plasma oder Serum aus der Blutprobe, die von dem Patienten, von dem vermutet wird oder von dem bekannt ist, dass er an dem Erkrankungszustand leidet, erhalten wurde,
(ii) Trennen des Plasmas oder Serums in zwei oder mehr Fraktionen so dass Partikel-assoziierte und nicht-Partikel-assoziierte Nukleinsäure in zwei verschiedenen Fraktionen vorhanden sind; und
(iii) Evaluieren des Erkrankungszustands durch Vergleichen der relativen Menge oder Konzentration oder Eigenschaft von Partikel-assoziierter und nicht-Partikel-assoziierter Nukleinsäure einer Nukleinsäurespezies in den Fraktionen und Vergleichen der relativen Menge oder Konzentration oder Eigenschaft der Nukleinsäure mit einer Kontrolle.

2. Verfahren nach Anspruch 1, wobei die Nukleinsäure eine mRNA oder eine DNA ist.

3. Verfahren nach Anspruch 2, wobei die mRNA ausgewählt ist aus der Gruppe von Glycerinaldehyd-3-phosphat-Dehydrogenase (GAPDH) mRNA und Plättchen-basischem-Protein mRNA.

4. Verfahren nach einem der voranstehenden Ansprüche, weiter umfassend den Schritt von Amplifzieren der Nukleinsäure.

5. Verfahren nach Anspruch 4, wobei die Nukleinsäure durch PCR oder reverse Transkriptase PCR amplifiziert wird.

6. Verfahren nach Anspruch 5, wobei die Nukleinsäure durch real-time PCR oder real-time reverse Transkriptase PCR amplifiziert wird.

7. Verfahren nach einem der voranstehenden Ansprüche, wobei die Fraktionen durch Filtration getrennt werden.

8. Verfahren nach Anspruch 7, wobei die Filtergröße ungefähr 5 µm oder kleiner ist.

9. Verfahren nach Anspruch 8, wobei die Filtergröße ungefähr t 0.22 µm ist.

10. Verfahren nach Anspruch 8, wobei die Filtergröße ungefähr 0.45 µm ist.

11. Verfahren nach einem der Ansprüche 1 to 6, wobei die Fraktionen durch Zentrifugation getrennt werden.

12. Verfahren nach Anspruch 11, wobei die Zentrifugation Ultrazentrifugation ist.

13. Verfahren nach einem der voranstehenden Ansprüche wobei der Erkrankungszustand ausgewählt ist aus der Gruppe bestehend aus Krebs, Schlaganfall und Trauma.

14. Verfahren nach Anspruch 13, wobei der Krebs hepatozellulärer Krebs oder nasopharyngeales Karzinom ist.

15. Verfahren nach einem der voranstehenden Ansprüche, wobei die Patientin schwanger ist und das Verfahren zum Evaluieren eines Erkrankungs- oder physiologischen Zustands in der Patientin oder ihrem Fötus beim Nachweis, der Überwachung, Prognose oder Behandlung der Patientin oder ihrem Fötus hilft.

## Revendications

1. Procédé permettant d'évaluer un état pathologique chez un patient suspecté de souffrir ou connu pour souffrir de l'état pathologique, ledit procédé comprenant :
(i) l'isolement du plasma ou du sérum à partir de l'échantillon de sang obtenu du patient suspecté de souffrir ou connu pour souffrir d'un état pathologique,
(ii) la séparation du plasma ou du sérum en deux fractions ou plus de telle manière que l'acide nucléique associé aux particules et l'acide nucléique non associé aux particules sont présents dans deux fractions différentes ; et
(iii) l'évaluation de l'état pathologique en comparant la quantité relative ou la concentration ou la caractéristique de l'acide nucléique associé aux particules et de l'acide nucléique non associé aux particules dans lesdites fractions, et en comparant la quantité relative ou la concentration ou la caractéristique de l'acide nucléique avec un contrôle.

2. Procédé selon la revendication 2, dans lequel l'acide nucléique est un ARNm ou un ADN.

3. Procédé selon la revendication 2, dans lequel l'ARNm est choisi dans le groupe constitué de l'ARNm de la glycéraldéhyde-3-phosphate déshydrogénase (GAPDH) et de l'ARNm de la protéine basique des plaquettes.

4. Procédé selon l'une quelconque des revendications précédentes, comprenant en outre l'étape consistant à amplifier l'acide nucléique.

5. Procédé selon la revendication 4, dans lequel l'acide nucléique est amplifié par PCR ou PCR après transcription inverse.

6. Procédé selon la revendication 5, dans lequel l'acide nucléique est amplifié par PCR en temps réel ou PCR après transcription inverse en temps réel.

7. Procédé selon l'une quelconque des revendications précédentes, dans lequel les fractions sont séparées par filtration.

8. Procédé selon la revendication 7, dans lequel la taille du filtre est d'environ 5 µm ou inférieure.

9. Procédé selon la revendication 8, dans lequel la taille du filtre est d'environ 0,22 µm.

10. Procédé selon la revendication 8, dans lequel la taille du filtre est d'environ 0,45 µm.

11. Procédé selon l'une quelconque des revendications 1 à 6, dans lequel les fractions sont séparées par centrifugation.

12. Procédé selon la revendication 11, dans lequel la centrifugation est une ultracentrifugation.

13. Procédé selon l'une quelconque des revendications précédentes, où l'état pathologique est choisi dans le groupe constitué d'un cancer, d'un accident vasculaire cérébral et d'un traumatisme.

14. Procédé selon la revendication 13, où le cancer est un cancer hépatocellulaire ou un carcinome rhinopharyngé.

15. Procédé selon l'une quelconque des revendications précédentes, ou la patiente est enceinte et le procédé d'évaluation d'un état pathologique ou physiologique chez la patiente ou son foetus aide à la détection, au contrôle, au pronostic ou au traitement de la patiente ou de son foetus.
